# EUROPEAN PATENT SPECIFICATION

(11) **EP 4 284 131 B1**
(45) Date of publication and mention of the grant of the patent: **26.08.2026**
(21) Application number: 23188747.2
(22) Date of filing: 17.01.2020
(51) Int. Cl.: A61B 5/00, A61B 1/00, A61B 1/313, A61B 46/10, H05K 7/14

(54) **MODULAR ENDOSCOPIC SYSTEM FOR VISUALIZATION OF DISEASE**
MODULARES ENDOSKOPISCHES SYSTEM ZUR VISUALISIERUNG VON KRANKHEITEN
SYSTÈME ENDOSCOPIQUE MODULAIRE POUR LA VISUALISATION D'UNE MALADIE

(30) Priority: 17.01.2019 US 201962793846 P; 04.06.2019 US 201962857183 P
(43) Date of publication of application: 29.11.2023
(62) Document number(s) of the earlier application(s) in accordance with Art. 76 EPC: 20740856.8 / 3 911 222
(73) Proprietor: SBI Alapharma Canada, Inc., Toronto, ON M5G 1Z8 (CA)
(72) Inventor: DACOSTA, Ralph, Toronto, M5G 1T6 (CA); GIBSON, Christopher, Toronto, M5G 2C4 (CA); OTTOLINO-PERRY, Kathryn, Etobicoke, M5G 2C4 (CA); ANANTHA, Nayana Thalanki, Toronto, M5G 2C4 (CA); TREADWELL, Simon, Mississauga, L4X 2V7 (CA); DAYNES, Todd, Toronto, M5G 1T6 (CA); MEANEY, Todd, Toronto, M5G 1T6 (CA); VERMEY, Garrett R., Toronto, M3K 3S1 (CA); ANNIS, Carl, Mississauga, L4X 2V7 (CA)
(74) Representative: PGA S.p.A., Milano, Succursale di Lugano

(56) References cited:
- EP-A1- 1 880 660
- US-A1- 2016 213 236
- US-A1- 2018 242 848
- US-B2- 6 571 119

## Description

### TECHNICAL FIELD

The present disclosure relates to devices, systems, and methods for visualization of disease and removal of disease including, for example, tumors. The disclosed devices, systems, and methods may also be used to stage tumors and to assess surgical margins such as tissue margins on excised tumors and margins on tissue beds/surgical beds within surgical cavities from which a tumor and/or tissue has been removed. The disclosed devices, systems, and methods may also be used to identify one or more of residual cancer cells, precancerous cells, and satellite lesions and to provide guidance for removal and/or treatment of the same. The disclosed systems may also be used to visualize wounds, particularly those in confined anatomical spaces such as body lumens, hollow organs during surgery, etc. The devices and systems may be used to assess and quantify the components present in the area being visualized, e.g., bacteria, disease, infection, etc. The systems and methods may be suitable for collecting data regarding biochemical, biological and/or non-biological substances. The data may include, for example, one or more of white light data, fluorescent data, thermal data, infrared data, such as in wound care, for both human and animal applications.

### INTRODUCTION

Surgery is one of the oldest types of cancer therapy and is an effective treatment for many types of cancer. Oncology surgery may take different forms, dependent upon the goals of the surgery. For example, oncology surgery may include biopsies to diagnose or determine a type or stage of cancer, tumor removal to remove some or all of a tumor or cancerous tissue, exploratory surgery to locate or identify a tumor or cancerous tissue, debulking surgery to reduce the size of or remove as much of a tumor as possible without adversely affecting other body structures, and palliative surgery to address conditions caused by a tumor such as pain or pressure on body organs.

In surgeries in which the goal is to remove the tumor(s) or cancerous tissue, surgeons often face uncertainty in determining if all cancer has been removed. The surgical bed, or tissue bed, from which a tumor is removed, may contain residual cancer cells, i.e., cancer cells that remain in the surgical margin of the area from which the tumor is removed. If these residual cancer cells remain in the body, the likelihood of recurrence and metastasis increases. Often, the suspected presence of the residual cancer cells, based on examination of surgical margins of the excised tissue during pathological analysis of the tumor, leads to a secondary surgery to remove additional tissue from the surgical margin.

For example, breast cancer, the most prevalent cancer in women, is commonly treated by breast conservation surgery (BCS), e.g., a lumpectomy, which removes the tumor while leaving as much healthy breast tissue as possible. Treatment efficacy of BCS depends on the complete removal of malignant tissue while leaving enough healthy breast tissue to ensure adequate breast reconstruction, which may be poor if too much breast tissue is removed. Visualizing tumor margins under standard white light (WL) operating room conditions is challenging due to low tumor-to-normal tissue contrast, resulting in reoperation (i.e., secondary surgery) in approximately 23% of patients with early stage invasive breast cancer and 36% of patients with ductal carcinoma *in situ.* Re-excision is associated with a greater risk of recurrence, poorer patient outcomes including reduced breast cosmesis and increased healthcare costs. Positive surgical margins (i.e., margins containing cancerous cells) following BCS are also associated with decreased disease specific survival.

Current best practice in BCS involves palpation and/or specimen radiography and rarely, intraoperative histopathology to guide resection. Specimen radiography evaluates excised tissue margins using x-ray images and intraoperative histopathology (touch-prep or frozen) evaluates small samples of specimen tissue for cancer cells, both of which are limited by the time delay they cause (^{~} 20 min) and inaccurate co-localization of a positive margin on the excised tissue to the surgical bed. Thus, there is an urgent clinical need for a real-time, intraoperative imaging technology to assess excised specimen and surgical bed margins and to provide guidance for removal of one or more of residual cancer cells, precancerous cells, and satellite lesions.

In addition, conventional clinical assessment methods of acute and chronic wounds continue to be suboptimal. They are usually based on a complete patient history, qualitative and subjective clinical assessment with simple visual appraisal using ambient white light and the 'naked eye', and can sometimes involve the use of color photography to capture the general appearance of a wound under white light illumination [Perednia (1991) J Am Acad Dermatol. 25: 89-108]. Regular reassessment of progress toward healing and appropriate modification of the intervention is also necessary. Wound assessment terminology is non-uniform, many questions surrounding wound assessment remain unanswered, agreement has yet to be reached on the key wound parameters to measure in clinical practice, and the accuracy and reliability of available wound assessment techniques vary. Visual assessment is frequently combined with swabbing and/or tissue biopsies for bacteriological culture for diagnosis. Bacterial swabs are collected at the time of wound examination and have the noted advantage of providing identification of specific bacterial/microbial species [Bowler, 2001; Cutting, 1994; Dow, 1999; Dow G. In: Krasner et al. eds. Chronic Wound Care: A Clinical Source Book for Healthcare Professionals, 3rd ed. Wayne Pa.: HMP Communications. 2001:343-356]. However, often, multiple swabs and/or biopsies are collected randomly from the wound site, and some swabbing techniques may in fact spread the microorganisms around with the wound during the collection process thus affecting patient healing time and morbidity [Dow, 1999]. This may be a problem especially with large chronic (nonhealing) wounds where the detection yield for bacterial presence using current swabbing and biopsy protocols is suboptimal (diagnostically insensitive), despite many swabs being collected. Thus, current methods for obtaining swabs or tissue biopsies from the wound site for subsequent bacteriological culture are based on a non-targeted or 'blind' swabbing or punch biopsy approach, and have not been optimized to minimize trauma to the wound or to maximize the diagnostic yield of the bacteriology tests.
US 6571119B2 discloses a system with an endoscope connected to a base unit by lines receiving light from light sources in the base unit and by a CCD cable communicating with processors also housed in the base unit. US2018242848A1 discloses a portable handheld device with a light sensor and light sources. The device has an endoscope portion.

### SUMMARY

A portable, handheld endoscopic imaging system according to the invention is disclosed in any one of appended claims 1-12.

### BRIEF DESCRIPTION OF THE DRAWINGS

The present disclosure can be understood from the following detailed description either alone or together with the accompanying drawings. The drawings are included to provide a further understanding and are incorporated in and constitute a part of this specification. The drawings illustrate one or more exemplary embodiments of the present disclosure and together with the description serve to explain various principles and operations.
FIG. 1A is an illustration of the conversion of ALA to PpIX in a tumor cell;
FIG. 1B shows peak absorption and emission for PpIX;
FIG. 2 is a user-facing (front) view of an endoscopic handheld imaging device for visualization of disease according to the present disclosure.
FIG. 3 is a front perspective view of the endoscopic handheld imaging device for visualization of disease of FIG. 2.
FIG. 4 is a rear perspective view of the endoscopic handheld imaging device for visualization of disease of FIG. 2
FIG. 5 is a rear (patient-facing) view of the handheld imaging device of FIG. 2.
FIG. 6 is a side view of the handheld imaging device of FIG. 2.
FIG. 7 shows an endoscopic housing portion and a base body portion of a modular embodiment of the endoscopic imaging device in a disconnected configuration according to the disclosure.
FIG. 8 is a cross-sectional view of the modular endoscopic handheld imaging device of FIG. 7.
FIG. 9 is a perspective view of the modular endoscopic imaging device of FIG. 7.
FIG. 10 is an end view of the endoscope of the modular device of FIG. 7 in a first position.
FIG. 11 is another end view of the endoscope of the modular device of FIG. 7 in a second position.
FIG. 12 is an end view of the endoscope of the modular device of FIG. 7 which identifies the visible components of the endoscopic end.
FIG. 13 is a view of a PCB board for the endoscope of the handheld imaging device.
FIG. 14 is a photograph of a prototype of an endoscopic handheld imaging device according to an embodiment of the present disclosure.
FIG. 15 is a photograph of the prototype of FIG. 14 with a surgical drape in accordance with the present disclosure.

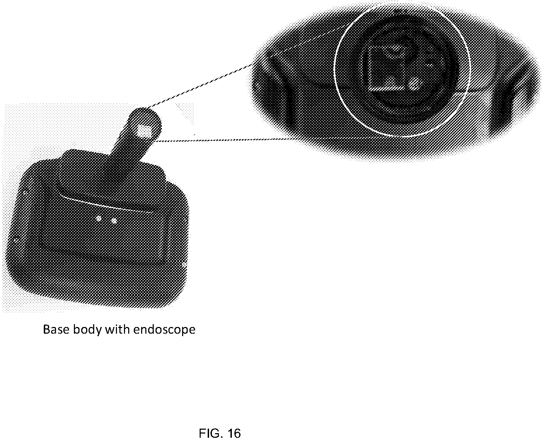

FIG. 16 is an enlarged view of the endoscopic end of the prototype of FIG. 14.

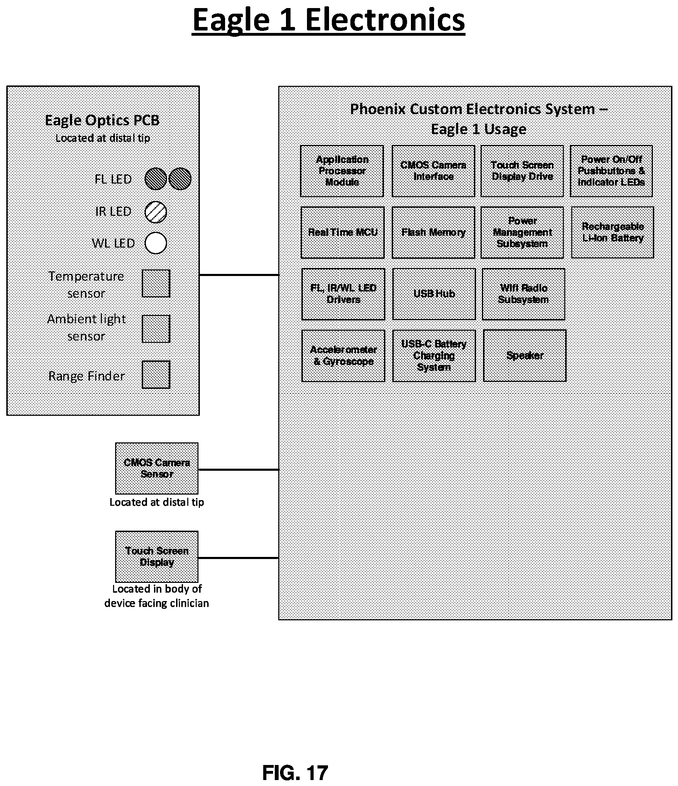

FIG. 17 is an exemplary schematic of an electronics configuration for an endoscopic imaging system in accordance with the present disclosure.
FIGS. 18-23 are charts showing exemplary bands of various filters configured to detect emissions excited by an excitation light and incorporated into embodiments of the handheld device according to the present disclosure.
FIG. 24 shows an embodiment of a handheld endoscopic imaging system which includes separate endoscopic optical heads for white light imaging, fluorescent imaging, and infrared imaging.
FIG. 25 shows the absorption and emission peaks for PpIX as relates to fluorescent imaging in accordance with one aspect of the present disclosure.
FIGS. 26A-26C are charts relating to characteristics of an embodiment of fluorescent LEDs used for fluorescent imaging in accordance with one aspect of the present disclosure.
FIGS. 27A-27C are charts relating to characteristics of an embodiment of a fluorescent emissions filter used for fluorescent imaging in accordance with one aspect of the present disclosure.
FIG. 28 shows the absorption and emission peaks for ICG dye, to be used with infrared imaging in accordance with one aspect of the present disclosure.
FIGS. 29A and 29B are charts relating to characteristics of an embodiment of infrared LEDs used for infrared imaging in accordance with one aspect of the present disclosure.
FIGS. 30A-30C are charts relating to characteristics of an embodiment of an infrared emissions filter used for infrared imaging in accordance with one aspect of the present disclosure.
FIG. 31 illustrates transmission characteristics of an embodiment of a lens of the handheld endoscopic imaging device.
FIGS. 32A-32C show various aspects of an embodiment of a custom sterile drape with lens cap for use with the handheld endoscopic imaging device in accordance with one aspect of the present disclosure.
FIG. 33 is a perspective view of a handheld endoscopic imaging device resting on a docking station according to an exemplary embodiment of the present disclosure.
FIG. 34 is a perspective view of the handheld endoscopic imaging device and docking station of FIG. 33 shown separated to illustrate other features of the docking station.
FIG. 35 is a front orthogonal view of the docking station of FIG. 33.
FIG. 36 is a schematic diagram showing various components of a handheld endoscopic imaging device according to an exemplary embodiment of the disclosure.
FIG. 37 is a perspective view of an interconnection cable according to an exemplary embodiment of the present disclosure.
FIG. 38 is a perspective view of a portion of a handheld endoscopic imaging device including an interconnection port according to an exemplary embodiment of the present disclosure.
FIG. 39 is perspective view of a lens of a sterile drape according to an exemplary embodiment of the present disclosure.
FIG. 40 is a cross-sectional side view of a handheld endoscopic imaging device with a sterile drape including the lens of FIG. 39 according to an exemplary embodiment of the present disclosure.
FIG. 41 is a perspective view of a darkening drape adaptor according to an exemplary embodiment of the present disclosure.
FIG. 42 is a perspective view of a handheld endoscopic imaging device with a darkening drape including the adaptor of FIG. 41 according to an embodiment of the present disclosure.

### DESCRIPTION OF VARIOUS EXEMPLARY EMBODIMENTS

Existing margin assessment technologies focus on the excised sample to determine whether surgical margins include residual cancer cells. These technologies are limited by their inability to accurately spatially co-localize a positive margin detected on the excised sample to the surgical bed, a limitation the present disclosure overcomes by directly imaging the surgical cavity. In addition to examining surgical margins, the disclosed devices and methods may be used to visualize surgical beds, visualize wounds, and access any confined space, such as organs, body lumens, etc. to visualize diseases other than cancer.

Other non-targeted techniques for reducing re-excisions include studies which combine untargeted margin shaving with standard of care BCS. While this technique may reduce the overall number of re-excisions, the approach includes several potential drawbacks. For example, larger resections are associated with poorer cosmetic outcomes and the untargeted removal of additional tissues is contradictory to the intention of BCS. In addition, the end result of using such a technique appears to be in conflict with the recently updated ASTRO/SSO guidelines, which defined positive margins as 'tumor at ink' and found no additional benefit of wider margins. Moran MS, Schnitt SJ, Giuliano AE, Harris JR, Khan SA, Horton J et al., "Society of Surgical Oncology-American Society for Radiation Oncology consensus guideline on margins for breast-conserving surgery with wholebreast irradiation in stages I and II invasive breast cancer," Ann Surg Oncol. 2014. 21(3):704-716. A recent retrospective study found no significant difference in re-excisions following cavity shaving relative to standard BCS. Pata G, Bartoli M, Bianchi A, Pasini M, Roncali S, Ragni F., "Additional Cavity Shaving at the Time of Breast-Conserving Surgery Enhances Accuracy of Margin Status Examination," Ann Surg Oncol. 2016. 23(9):2802-2808. Should margin shaving ultimately be found effective, FL-guided surgery may be used to refine the process by adding the ability to target specific areas in a surgical margin for shaving, thus turning an untargeted approach, which indiscriminately removes additional tissue, into a targeted approach that is more in line with the intent of BCS.

The present application discloses devices, systems, and methods for fluorescent-based visualization of tumors, including *ex vivo, in vivo* and *in vitro* visualization and/or assessment of tumors, multifocal disease, surgical beds and surgical margins, and intraoperative guidance for removal of residual tumor, satellite lesions, precancerous cells, and/or cancer cells in surgical margins. In certain embodiments, the devices disclosed herein are handheld and are configured to be at least partially positioned within a surgical cavity. In other embodiments, the devices are portable, without wired connections. However, it is within the scope of the present disclosure that the devices may be larger than a handheld device, and instead may include a handheld component. In such embodiments, it is contemplated that the handheld component may be connected to a larger device housing or system by a wired connection.

Also disclosed are methods for intraoperative, *in-vivo* imaging using the device and/or system. The imaging device may be multispectral. It is also contemplated that the device may be hyperspectral. In addition to providing information regarding the type of cells contained within a surgical margin, the disclosed devices and systems also provide information regarding location (i.e., anatomical context) of cells contained within a surgical margin. In addition, methods of providing guidance for intraoperative treatment of surgical margins using the device are disclosed, for example, fluorescence-based image guidance of resection of a surgical margin. The devices, systems, and methods disclosed herein may be used on subjects that include humans and animals.

In accordance with one aspect of the present disclosure, some disclosed methods combine use of the disclosed devices and/or systems with administration of a non-activated, non-targeted compound configured to induce porphyrin in tumor/cancer cells, precancer cells, and/or satellite lesions. For example, the subject may be given a diagnostic dose (i.e., not a therapeutic dose) of a compound (imaging/contrast agent) such as the pro-drug aminolevulinic acid (ALA). As understood by those of ordinary skill in the art, dosages of ALA less than 60 mg/kg are generally considered diagnostic while dosages greater than 60 mg/kg are generally considered therapeutic. As disclosed herein, the diagnostic dosage of ALA may be greater than 0 mg/kg and less than 60 kg/mg, between about 10 mg/kg and about 50 mg/kg, between about 20 mg/kg and 40 mg/kg, and may be administered to the subject in a dosage of 5 mg/kg, 10 mg/kg, 15 kg/mg, 20 mg/kg, 25 mg/kg, 30 mg/kg, 35 mg/kg, 40 mg/kg, 45 mg/kg, 50 mg/kg, or 55 mg/kg. The ALA may be administered orally, intravenously, via aerosol, via immersion, via lavage, and/or topically. Although a diagnostic dosage is contemplated for visualization of the residual cancer cells, precancer cells, and satellite lesions, it is within the scope of the present disclosure to use the disclosed devices, systems, and methods to provide guidance during treatment and/or removal of these cells and/or lesions. In such a case, the surgeon's preferred method of treatment may vary based on the preferences of the individual surgeon. Such treatments may include, for example, photodynamic therapy (PDT). In cases where PDT or other light-based therapies are contemplated as a possibility, administration of a higher dosage of ALA, i.e., a therapeutic dosage rather than a diagnostic dosage, may be desirable. In these cases, the subject may be prescribed a dosage of ALA higher than 60 mg/kg.

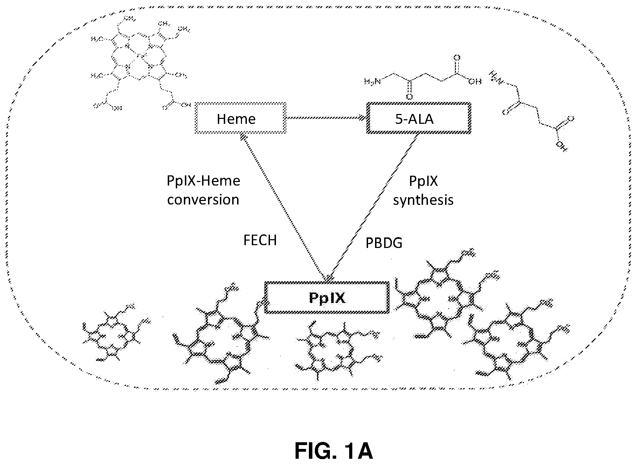

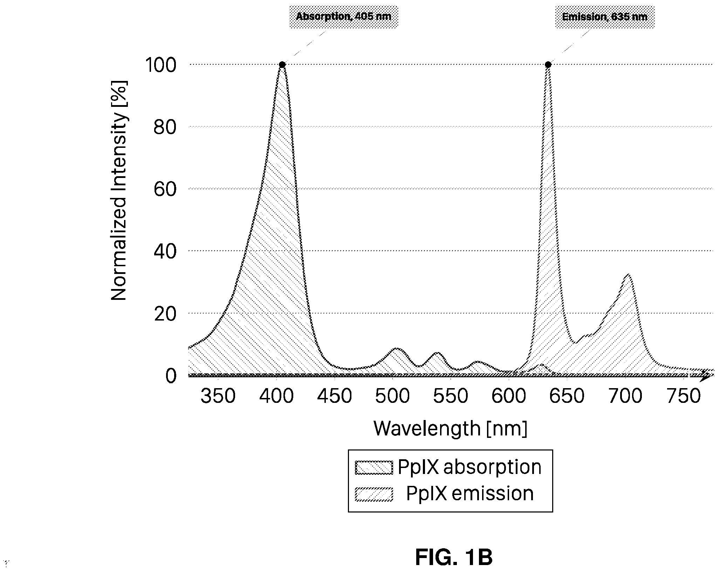

The ALA induces porphyrin formation (protoporphyrin IX (PpIX)) in tumor/cancer cells which when excited by the appropriate excitation light, results in a red fluorescence emission from cells containing the PpIX, which enhances the redto-green fluorescence contrast between the tumor/cancer tissue cells and normal tissue cells (e.g., collagen) imaged with the device. ALA is non-fluorescent by itself, but PpIX is fluorescent at around 630 nm, 680 nm, and 710 nm, with the 630 nm emission being the strongest. FIG. 1B illustrates the fluorescence emission of PpIX when excited with excitation light having a wavelength of 405 nm. Alternatively, the endogenous fluorescent difference between tumor/cancer cells or precancer cells and normal/healthy cells may be used without an imaging/contrast agent.

In exemplary embodiments, the non-activated, non-targeted compound configured to induce porphyrin in tumor/cancer cells, precancer cells, and/or satellite lesions is administered to a subject between about 15 minutes and about 6 hours before surgery, about 1 hour and about 5 hours before surgery, between about 2 hours and about 4 hours before surgery, or between about 2.5 hours and about 3.5 hours before surgery. These exemplary time frames allow sufficient time for the ALA to be converted to porphyrins in tumor/cancer cells, precancer cells, and/or satellite lesions. The ALA or other suitable compound may be administered orally, intravenously, via aerosol, via immersion, via lavage, and/or topically.

In cases where the administration of the compound is outside of the desired or preferred time frame, it is possible that PpIX may be further induced (or induced for the first time if the compound was not administered prior to surgery) by, for example, applying the compound via an aerosol composition, i.e., spraying it into the surgical cavity or onto the excised tissue (before or after sectioning for examination). Additionally or alternatively, the compound may be administered in a liquid form, for example as a lavage of the surgical cavity. Additionally or alternatively, with respect to the removed specimen, PpIX may be induced in the excised specimen if it is immersed in the liquid compound, such as liquid ALA, almost immediately after excision. The sooner the excised tissue is immersed, the better the chance that PpIX or additional PpIX will be induced in the excised tissue.

During surgery, the tumor is removed by the surgeon, if possible. The handheld, fluorescence-based imaging device is then used to identify, locate, and guide treatment of any residual cancer cells, precancer cells, and/or satellite lesions in the surgical bed from which the tumor has been removed. The device may also be used to examine the excised tumor/tissue specimen to determine if any tumor/cancer cells and/or precancer cells are present on the outer margin of the excised specimen. The presence of such cells may indicate a positive margin, to be considered by the surgeon in determining whether further resection of the surgical bed is to be performed. The location of any tumor/cancer cells identified on the outer margin of the excised specimen can be used to identify a corresponding location on the surgical bed, which may be targeted for further resection and/or treatment. This may be particularly useful in situations in which visualization of the surgical bed itself does not identify any residual tumor/cancer cells, precancer cells, or satellite lesions.

In accordance with one aspect of the present disclosure, a handheld, endoscopic fluorescence-based imaging device for visualization of disease is provided. This device and method may be suitable for monitoring of wounds in humans and animals.

The device may be a modular handheld imaging device. The device comprises a base body portion, also referred to herein as a base portion or a base housing. The device also comprises an endoscopic optical portion, also referred to herein as an endoscopic optical housing. The endoscopic optical portion is releasably received by the base body portion and is interchangeable with other optical portions depending upon the capabilities desired for imaging in a given situation.

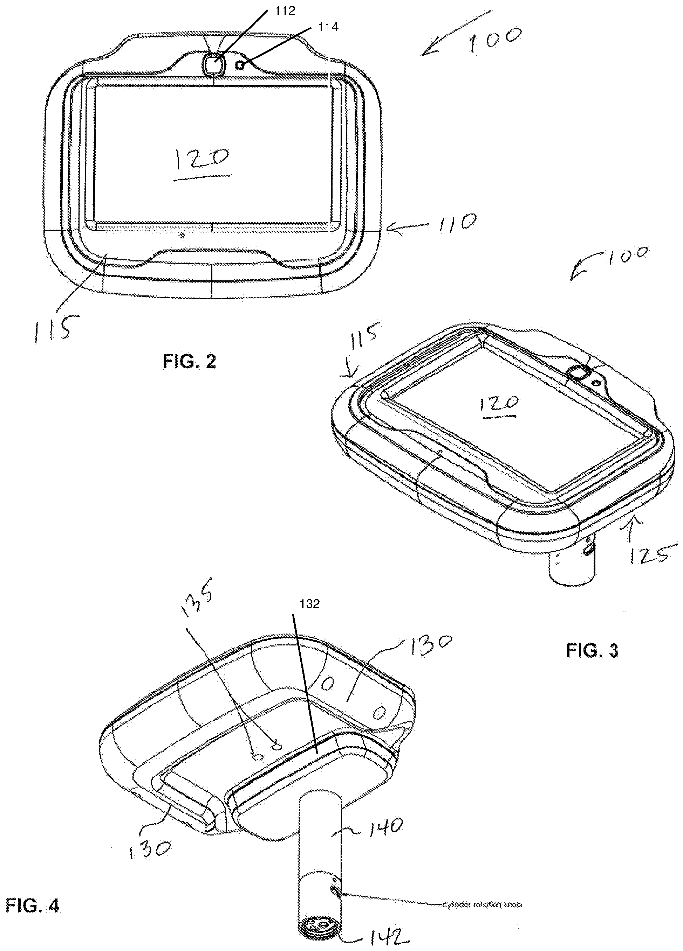

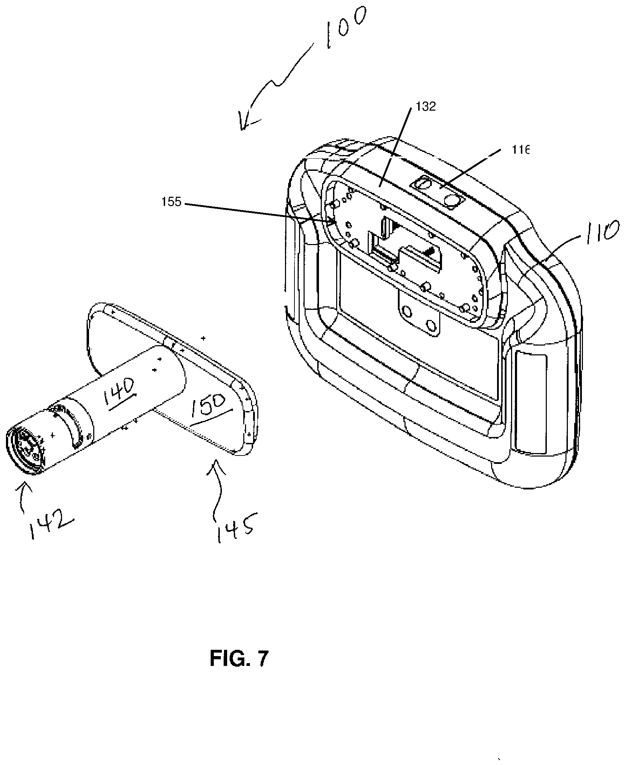

An exemplary embodiment of the handheld endoscopic imaging device 100 is shown in FIGS. 2-6. As shown in FIGS. 2-6, in some example embodiments, a base body portion 110 of device 100 may have a generally square or rectangular shape. A front, or user-facing side 115 of the base body portion 110 includes a display screen 120 for displaying images and videos captured by the device. In one example embodiment, the touchscreen display has a protective glass, including an anti-reflective coating, over top of it. Although depicted as square or rectangular, the device may take on any shape that will reasonably support a display screen such as a touchscreen. In addition to disclosing images captured by the imaging device 100, the display screen also operates as a user interface, allowing the user to control functions of the device via touchscreen input. Positioned above the display screen 120 may be a power button 112. In one example embodiment, a ring of LEDs may surround the power button and are used to indicate the system status. In the same area in which power button 112 is located, a battery status LED 114 may be provided. On a top surface of the device, a universal serial bus (USB) port (not shown) may be provided to facilitate connection of the handheld device 100 to an external device, such as a computer (such as a PC workstation), a tablet, a phone or other device for various tasks such as updating software or firmware on the handheld device 100, downloading images saved on memory of the handheld device 100, etc. USB port (not shown) may be protected by a cover 116 (see FIG. 7). Additionally or alternatively, the USB port of the handheld device 100 can be directly connected to a storage device (not shown), such as a USB stick, USB flash drive or thumb drive, to directly transfer data. For example, the handheld device 100 can download data, images, and/or other material to the storage device. In another example, the storage device can be used to load new software or instructions onto the handheld device. Alternatively, such actions can be accomplished with a wireless communication link between the handheld device 100 and an external device such as computer, tablet, phone or other device. In some embodiments, the handheld device 100 can include both a USB connection and wireless communication (such as Wi-Fi, Bluetooth, etc.) functionality.

Positioned on an opposite side of the device, on the patient-facing side 125 of the device, may be handhold areas 130 configured to facilitate a user holding the device during imaging. As illustrated in FIG. 4, the handhold areas may comprise protrusions or areas that extend away from the base body portion 110 sufficiently to allow a user's fingers to grip or wrap around the protrusions. Various other types of handholds as well as positioning of the handholds may be used. One consideration in the position of such handholds is the ability of the user to balance the device while using the device for imaging and while inputting commands via the touchscreen display. Weight distribution of the device will also be a consideration to provide a user-friendly and ergonomic device. The patient facing side 125 of the device may also incorporate contacts 135 for wireless charging of the device.

The patient-facing side 125 of device 100 also includes an endoscope 140. Endoscope 140 may be mounted on and/or integrally formed with endoscopic housing portion 145. As illustrated in the exemplary embodiment of FIG. 7, the endoscopic housing portion 145 may be detachable from base body portion 110. Endoscopic housing portion 145 includes a base 150 which supports the endoscope 140. Although illustrated as a rectangular base, it is contemplated that base 150 may take on other shapes such as square, circular, or oval. The base 150 is shaped and configured to be received in an opening 155 on the base body portion 110. The opening 155 is configured to releasably receive the endoscopic optical housing 145. When the base 150 of endoscopic housing portion 150 is positioned in opening 155, it may be locked into position such that endoscopic housing portion 145 is locked to base body portion 110. In this configuration, electrical contacts are made between base body portion 110 and the optical components contained in endoscopic housing portion 145 and the components in the endoscopic housing portion are powered by a power source, such as a battery, contained in the base body portion 110. The battery pack (not shown) may be located in a base area of the base body portion 110. In various example embodiments, the base body portion 110 includes a heat sink to dissipate heat from the device electronics. In one example embodiment, the heat sink 132 (FIG. 4) forms a lip or wall around the opening 155 in the base body portion 110 that is configured to receive the endoscopic optical housing. Additionally, the endoscopic housing portion 145 may be made of a material configured to pull heat away from the optics found on the distal tip of the endoscopic portion, such as for example, aluminum. The base body portion also may contain a Wi-Fi antenna (not shown) to permit the handheld device 100 to send and/or receive wireless communications. In one example embodiment, the Wi-Fi antenna is positioned near the outer shell or housing of base body portion 110 to maintain the Wi-Fi performance.

The base body portion 110 includes an interface configured to releasably receive the endoscopic optical housing portion 145. The endoscopic optical housing includes a portion configured to be received into the base body in a manner that provides electrical and power connections between the components in the endoscopic optical housing and the battery and processor in the base body portion as described below.

Systems according to the present disclosure can include a docking station, such as a stand, on which the handheld device can rest when not in use. The docking station can include one or more electrical connection areas, such as electrical contacts configured to provide charging current to a battery of the handheld device. The docking station can also include data connections such as one or more ports (e.g., USB ports) to connect the docking station to a workstation such as a computer.

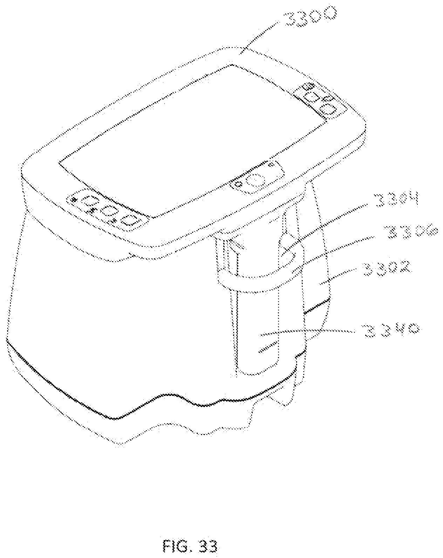

For example, referring now to FIG. 33, an embodiment of a handheld device 3300 and a docking station 3302 is shown. In FIG. 33, the handheld device 3300 is resting on the docking station 3302. The docking station 3302 includes a receptacle 3304 configured to receive an endoscope portion 3340 of the handheld device 3300. The docking station 3302 can include various features configured to provide secure placement of the handheld device 3300 on the docking station 3302. For example, in this embodiment, the docking station 3302 includes a retainer loop 3306 positioned to at least partly encircle the endoscope portion 3340 when the handheld device 3300 is placed on the docking station 3302 and ensure that inadvertent contact against the handheld device 3300 does not dislodge the handheld device 3300 from the docking station 3302. The docking station 3302 can also include rubber feet (not shown) to ensure the docking station 3302 has sufficient grip on a surface such as a counter or table on which the docking station is placed.

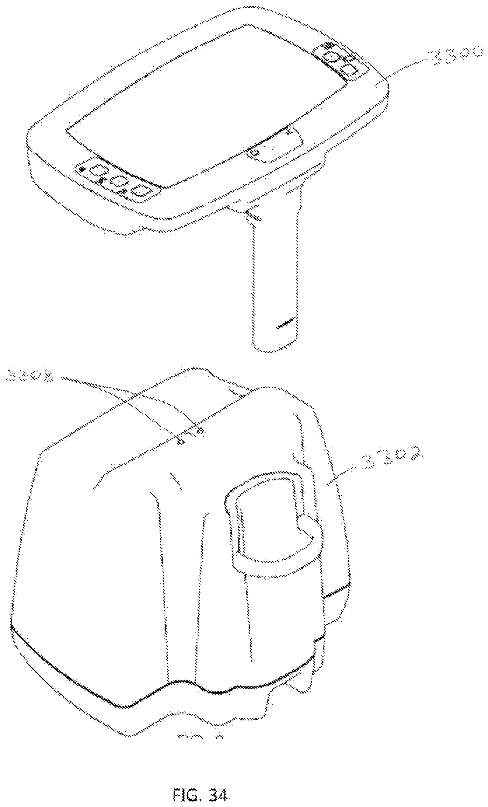

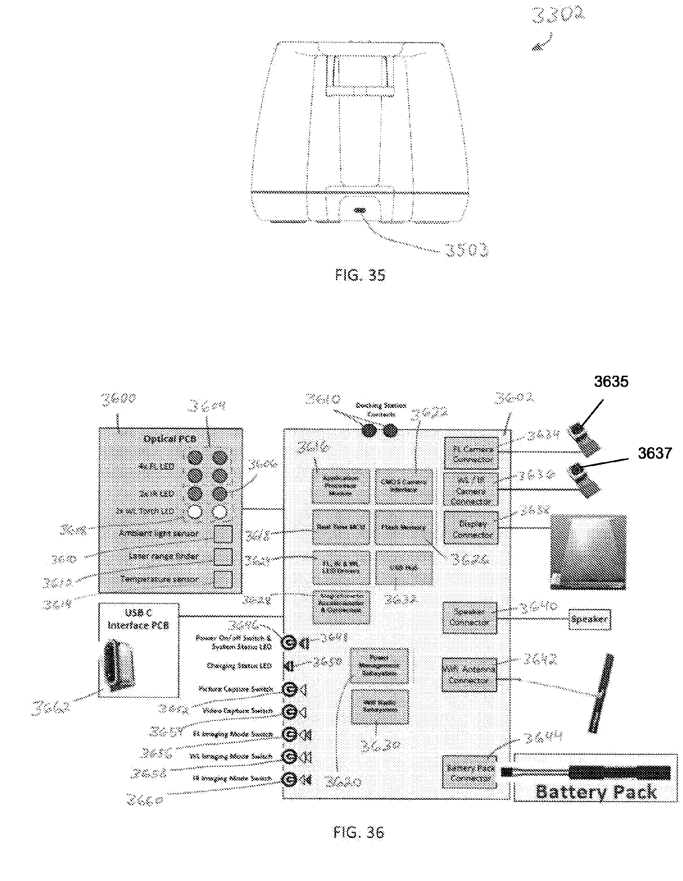

Referring now to FIG. 34, the handheld device 3300 and docking station 3302 are shown separated from one another. In FIG. 34, a pair of electrical contacts 3308 are visible on the docking station 3302. The handheld device 3300 can include corresponding contacts 3310 (shown schematically in FIG. 36) that touch the contacts 3308 when the handheld device 3300 is placed on the docking station 3302 to form an electrical connection between the handheld device 3300 and the docking station 3302 to provide an electrical pathway for charging the battery of the handheld device. Optionally, the contacts 3308 and 3310 (FIG. 36) can form an interface across which data can be transferred to or from the handheld device.

Referring now to FIG. 35, a front orthogonal view of the docking station 3302 is shown. In the view of FIG. 35, a universal serial bus (USB) port 3503 is visible. The USB port 3503 can be used to connect the docking station 3302 to a power source for charging the battery of the handheld device 3300 (FIG. 33), such as by connecting the docking station 3302 to a computer or AC power adaptor. The USB port 3503 can be or include, without limitation, mini-USB, micro-USB, USB type-C, or other standard USB connector configurations. In other embodiments, other types of connector devices for connecting the docking station 3302 to a power source are considered within the scope of the disclosure, such as other types of AC/DC converters or other types of power supplies. Alternatively, the docking station can include a built-in AC/DC converter and be provided with a power cable configured to be plugged into an AC main line, such as a traditional wall outlet supplying alternating current power.

The endoscopic optical housing may include various optical components configured to facilitate the collection of optical signals from a target being visualized. The properties that may vary from one optical housing to another include the following non-limiting examples, which may be included in any combination in each optical housing: number of image sensors, number of image sensors configured for white light imaging (i.e., combined with filter for white light imaging); number of image sensors configured for fluorescent imaging, wherein different images sensors for fluorescent imaging may be paired with different filters to permit passage of different ranges of fluorescent emissions, wherein each range is configured to capture a particular characteristic of a target (e.g., vasculature or microvasculature, collagen, elastin, blood, bone, bacteria, malignancy, healthy or diseased cartilage, ligaments, tendons, connective tissue, lymphatics, nerve, muscle etc.).

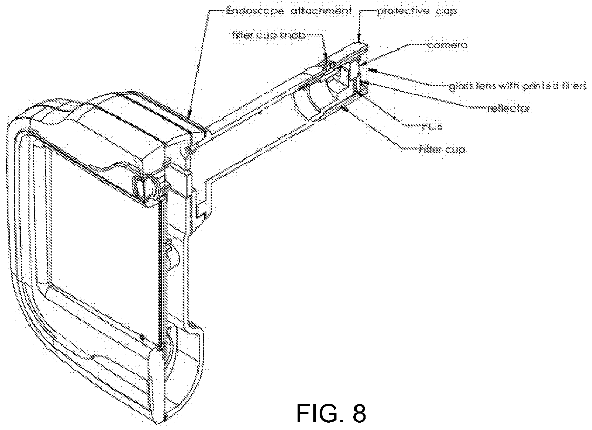

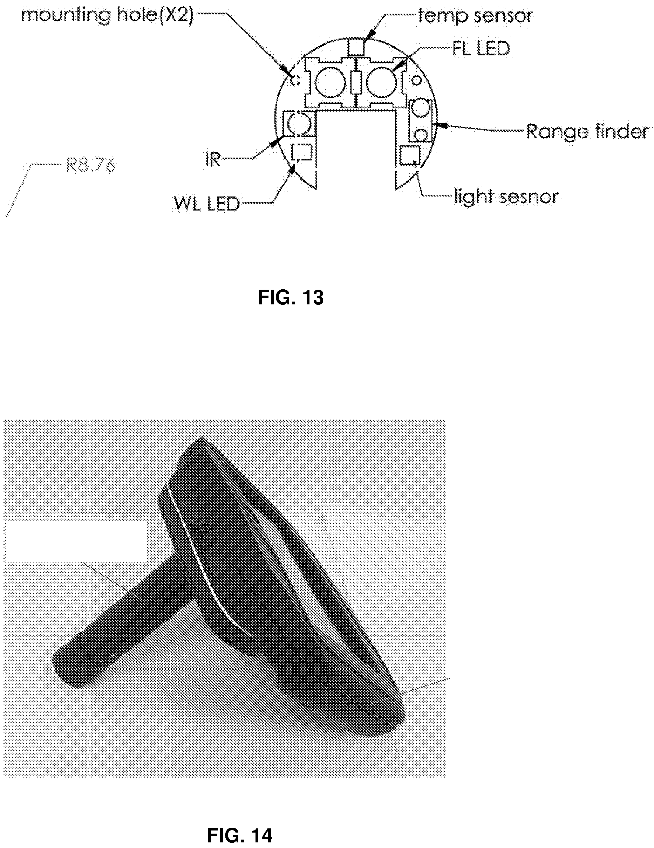

In the example embodiment illustrated in FIGS. 8-13, electrical components are located at the distal tip of endoscope 140 may include: one or more fluorescent excitation light sources (FL LEDs 160); a white light source (WL LED 165); an infrared source (IR LED 170); a digital temperature sensor 180, a digital range finder 175, and a digital ambient light sensor 185. The fluorescent excitation light sources and white light source may be embodied as LEDs and have wires to the LED cathode and anode terminals. The digital sensors may be powered from a DC regulator located in the base body. The microcontroller unit (MCU) in the base body 110 communicates with the sensors through I2C (an industry standard communication scheme). A camera sensor 190 is also provided in the distal tip 142 of the endoscope 140. The camera sensor 190 connects to an MCU located in the base body 110 using MIPI CSI (an industry standard camera interface scheme). While the exemplary embodiment of FIGS. 8-13 is illustrated with a single camera sensor 190, other exemplary embodiments can optionally include two or more camera sensors, as noted below. The endoscope may also include built-in polarization optics for reflectance and fluorescence polarization imaging.

The image sensor is configured to capture still images or video. Although disclosed herein as containing a single image sensor, it is contemplated that a different endoscopic design could include more than one image sensor. As a non-limiting example, a device according to the present disclosure may include a first camera sensor configured for white light and infrared imaging, and a second camera sensor configured for fluorescent imaging.

As will be understood by those of skill in the art, the arrangement of the components in the distal end of the imaging device may take on many configurations. Such configurations may be driven by size of the device, the footprint of the device, and the number of components used. However, when arranging the components, functional factors should also be taken into account. For example, issues such as light leakage from light sources of the device and/or an ambient light entering the optical housing at the distal end may interfere with proper or optimal operation of the device, and may for example cause a less desirable output, such as image artifacts. The arrangement illustrated in FIGS. 8-13 is one possible arrangement in which camera sensors are isolated so as to prevent light leakage from light sources and ambient light.

It may be desirable to have different components in the endoscope depending on the type of imaging to be done or the type of characteristics to be captured during imaging. Thus, it is possible that several different versions of endoscopic optical housings may be provided for use with a single base body 110. Each different endoscopic housing may contain a different combination of optical components, the specific combinations each tailored to create images depicting specific characteristics of the site being imaged. Depending on the number of type of components included in the distal tip 142 of endoscope 140, as well as the intended use of a particular endoscope, the size, shape, or materials of the endoscope may change. Thus, it is contemplated that the base body portion 110 may interchangeably receive several different endoscopic portions, each having different characteristics.

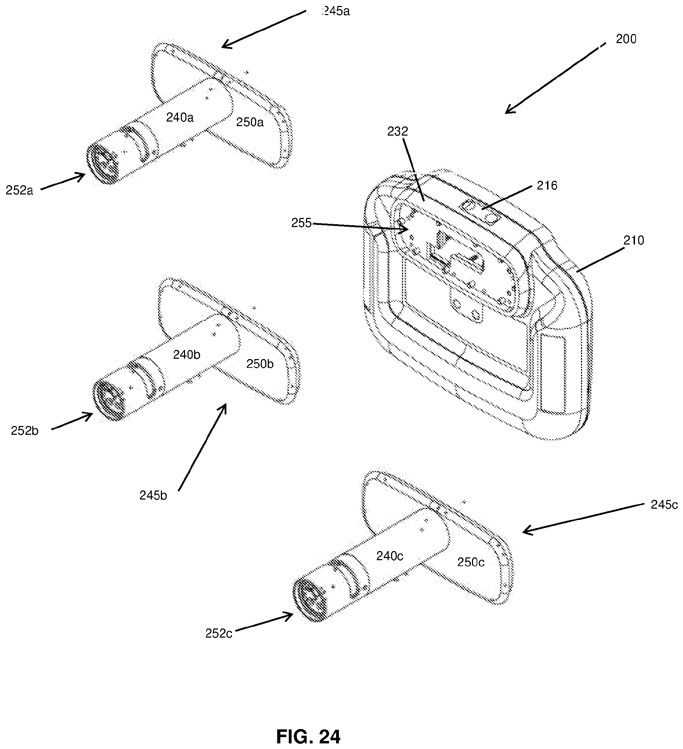

For example, in accordance with one example embodiment, each type of light source and/or imaging mode may be provided in a separate endoscopic housing. For example, as shown in FIG. 24, handheld endoscopic imaging device 200 includes a base body 210 and three endoscopic optical heads (imaging heads) 245a, 245b, and 245c. Each endoscopic imaging head is configured for a specific imaging mode. In the illustrated embodiment, a white light endoscopic optical head 245a is configured for a white light imaging mode. The distal tip 252a of endoscope 240a includes one or more white light LEDs and an optical sensor configured to receive white light images. The white light endoscopic optical head 245a is received in the opening 255 of base body 210 and, when white light endoscopic optical head 245a is operably connected to base body 210, the handheld endoscopic imaging device 200 is configured for a WL imaging mode. WL imaging illuminates the entire field of view (FOV) for viewing and capturing images of breast tissue under standard lighting conditions, similar to that present in an operating room setting. In one example embodiment, a WL endoscopic housing includes a plurality of white light LEDs and an image sensor, such as an Omni Vision sensor OV8865, with a resolution of 8 MP, a Focus Range of 2 - 15 cm, a Focal length of 3.05 mm, an F number 2.2, an Angle-of-view of 85°, and a TV Distortion of <1.0%. In another example embodiment, a WL endoscopic housing includes a plurality of white light LEDs and an image sensor, such as a Sony sensor IMX219, with a resolution of 8 MP, a Focus Range of 2 - 15 cm, a Focal length of 2.5 mm, an F number 2.2, an Angle-of-view of 84°, and a TV Distortion of <1.5%. The white light imaging endoscopic optical head 245a may also include additional components, such as a temperature sensor, a light sensor, and/or a rangefinder.

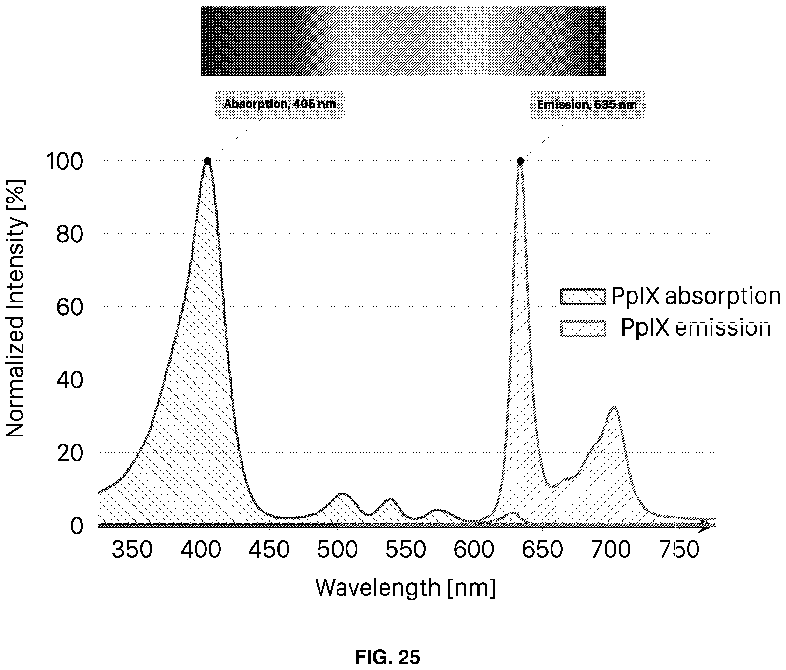

FIG. 24 further shows a fluorescent imaging endoscopic optical head 245b is configured for a fluorescent imaging mode. The distal tip 252b of endoscope 240b includes one or more fluorescent LEDs configured to emit excitation light and illuminate a target and an optical sensor configured to receive optical signals responsive to illumination of the target with the excitation light. The fluorescent endoscopic optical head 245b is received in the opening 255 of base body 210 and, when fluorescent imaging endoscopic optical head 245b is operably connected to base body 210, the handheld endoscopic imaging device 200 is configured for a fluorescent (FL) imaging mode. In one example embodiment, a fluorescent imaging endoscopic optical head includes a plurality of blue/violet LEDs and an image sensor, such as an Omni Vision sensor OV8865, with a resolution of 8 MP, a Focus Range of 2 - 15 cm, a Focal length of 3.05 mm, an F number 2.2, an Angle-of-view of 85°, and a TV Distortion of <1.0%. In another example embodiment, a fluorescent imaging endoscopic optical head includes a plurality of blue/violet LEDs and an image sensor, such as a Sony sensor IMX219, with a resolution of 8 MP, a Focus Range of 2 - 15 cm, a Focal length of 2.5 mm, an F number 2.2, an Angle-of-view of 84°, and a TV Distortion of <1.5%. The purpose of the FL imaging is to visualize carcinoma in breast tissue. Carcinoma in breast tissue is visualized by illuminating breast tissue with violet light (405 nm) to excite protoporphyrin (PpIX) that has accumulated within cancerous cells following the ingestion of 5-aminolevulinic acid (ALA) by the patient. The localized PpIX within the cancerous tumors absorb the excitation light (405 nm) and then emits light at a longer wavelength (peak at 635 nm) allowing for the visualization of carcinoma in breast tissue. See, for example, FIG. 25.

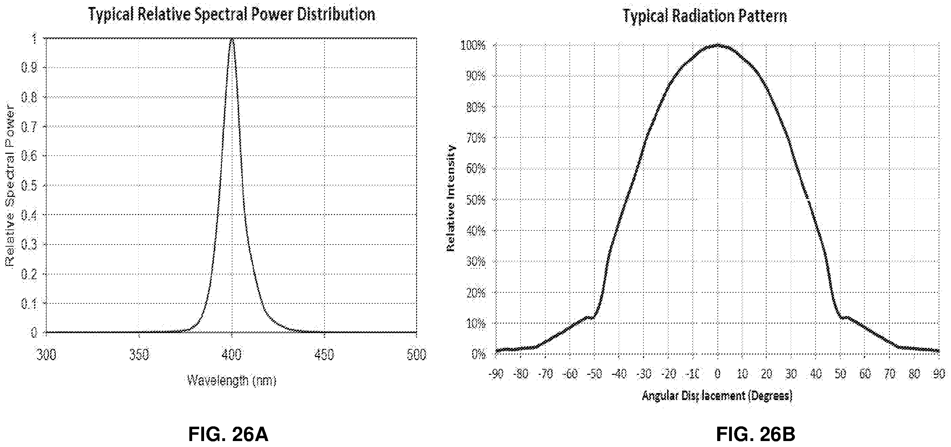

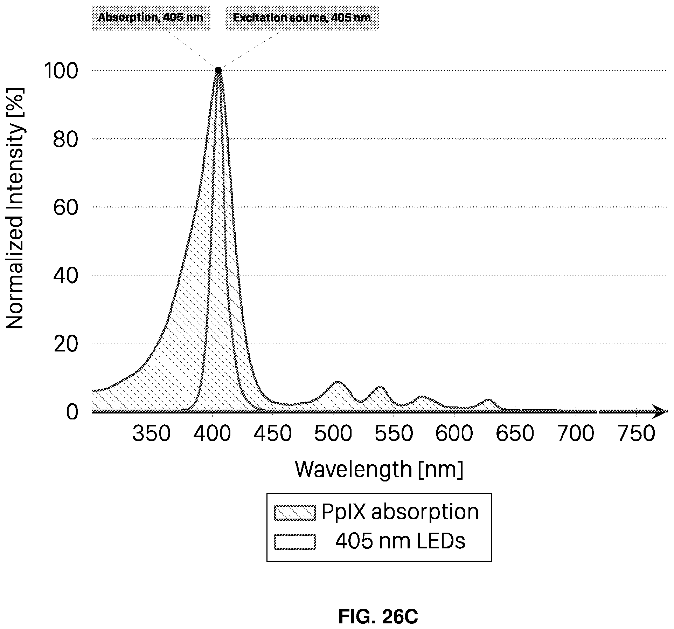

In one example embodiment, the fluorescent imaging endoscopic optical head 245b for use with handheld endoscopic imaging device 200 has two violet LED illumination sources that emit excitation light having a 405 nm ± 15 nm bandwidth at FWHM for inducing PpIX fluorescence. See FIGS. 26A-26C. The fluorescent imaging endoscopic optical head 245b for use with handheld endoscopic imaging device 200 may have 4 different power settings for the fluorescent illumination sources: low, low-mid, high-mid and high. In one example embodiment, the irradiance of the four settings when the distal end 245b of endoscopic imaging device is 10 cm away from the imaging target are low: about 2.3 mW/cm², low-mid: about 5.2 mW/cm², high-mid: about 8.1 mW/cm², and high: about 11.0 mW/cm².

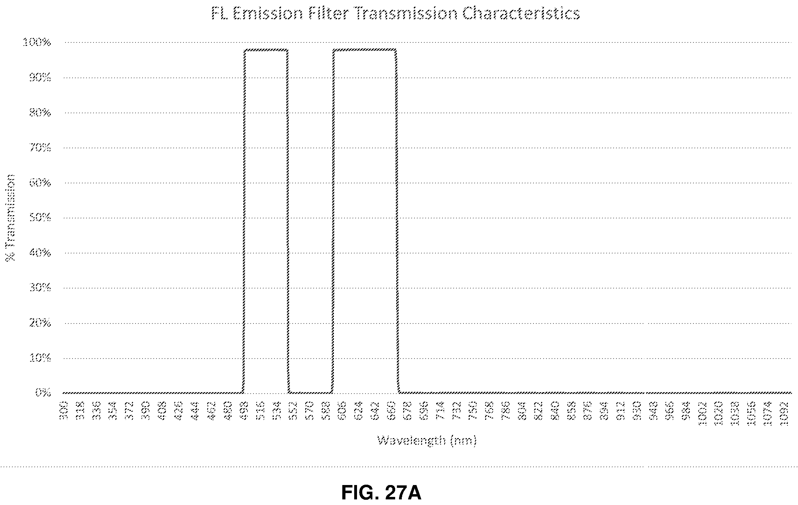

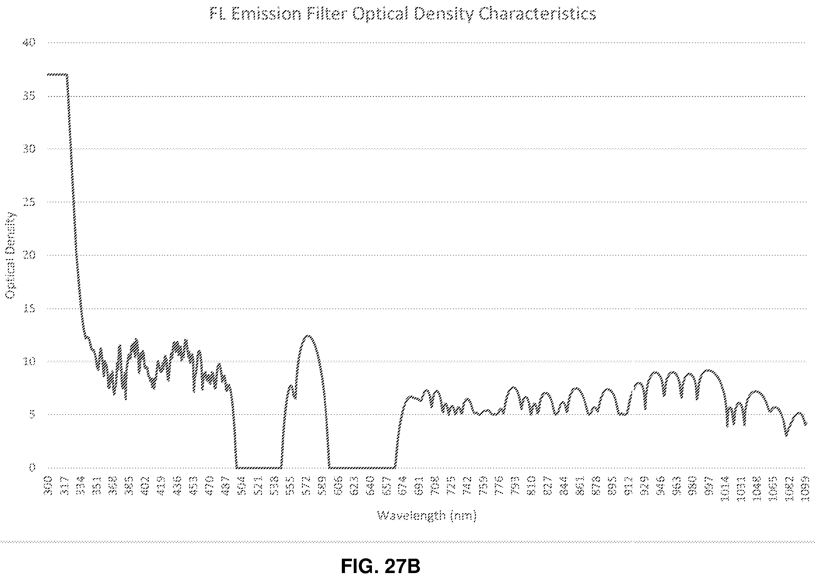

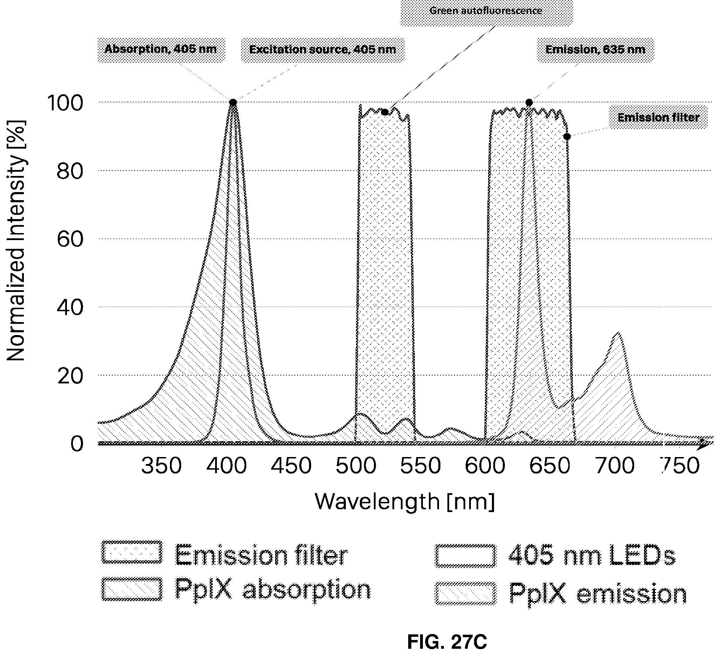

The fluorescent imaging endoscopic optical head 245b for use with handheld endoscopic imaging device 200 also includes a filter configured to permit passage of signals, responsive to illumination of the target with the FL excitation light, to the optical sensor (camera). In one example, the fluorescent imaging endoscopic optical head has a fixed dual-bandpass emissions filter for visualizing the PpIX emission light (635 nm) and the green autofluorescence from connective tissue (500 - 550 nm). In one example embodiment, the dual-bandpass emission filter has the following characteristics: diameter: 6 mm (+/0.1mm), thickness: 1 mm (+/0.1mm), average transmission greater 95% for the following emission ranges: 502 nm - 543 nm and 600 nm - 660 nm. The dual-bandpass emission filter is also configured to completely block (absolute block) the following wavelengths of light: 300 nm - 492 nm and 553 nm - 589 nm. The dual-bandpass emission filter is also configured to block average 675nm - 1000 nm and 1000 - 1200 nm wavelengths of light. The characteristics of this example fixed dual-bandpass emissions filter are illustrated in FIGS. 27A, 27B, and 27C.

The fluorescent imaging endoscopic optical head 245b may also include additional components, such as a temperature sensor, an ambient light sensor, and/or a rangefinder.

FIG. 24 further shows an infrared (IR) imaging endoscopic optical head 245c configured for an infrared imaging mode. The distal tip 252c of endoscope 240c includes one or more infrared LEDs configured to emit excitation light and illuminate a target and an optical sensor configured to receive optical signals responsive to illumination of the target with the excitation light. The infrared endoscopic optical head 245c is received in the opening 255 of base body 210 and, when infrared imaging endoscopic optical head 245c is operably connected to base body 210, the handheld endoscopic imaging device 200 is configured for an infrared (IR) imaging mode. In one example embodiment, an infrared imaging endoscopic optical head includes one or more infrared LEDs and an image sensor, such as an Omni Vision sensor OV8865, with a resolution of 8 MP, a Focus Range of 2 - 15 cm, a Focal length of 3.05 mm, an F number 2.2, an Angle-of-view of 85°, and a TV Distortion of <1.0%. In another example embodiment, an infrared imaging endoscopic optical head includes one or more infrared LEDs and an image sensor, such as a Sony sensor IMX219, with a resolution of 8 MP, a Focus Range of 2 - 15 cm, a Focal length of 2.5 mm, an F number 2.2, an Angle-of-view of 84°, and a TV Distortion of <1.5%.

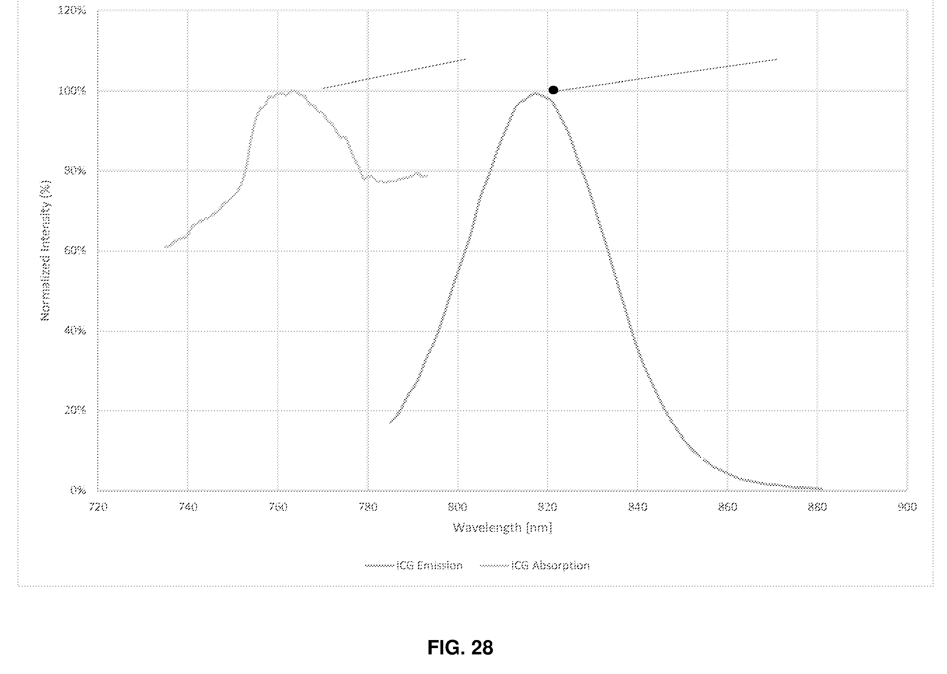

IR imaging in combination with the use of Indocyanine green (ICG) dye permits visualization of biological structures such as lymph nodes or blood vessels during breast conserving surgery. ICG is a cyanine dye administered to patients intravenously, it binds tightly to β-lipoproteins and particularly albumins. Albumins are a family of globular proteins and they are commonly found in the blood plasma and the circulatory system. Additionally, because of the high protein content of lymph nodes, ICG accumulates in the lymphatic pathways and lymph nodes. The accumulation of ICG makes visualizing lymph nodes and vasculature using IR imaging possible. ICG is a dye which fluoresces after excitation under near-infrared light with a peak absorption at 763 nm and a peak emission at 817 nm, as measured in a 60uM aqueous solution. See FIG. 28.

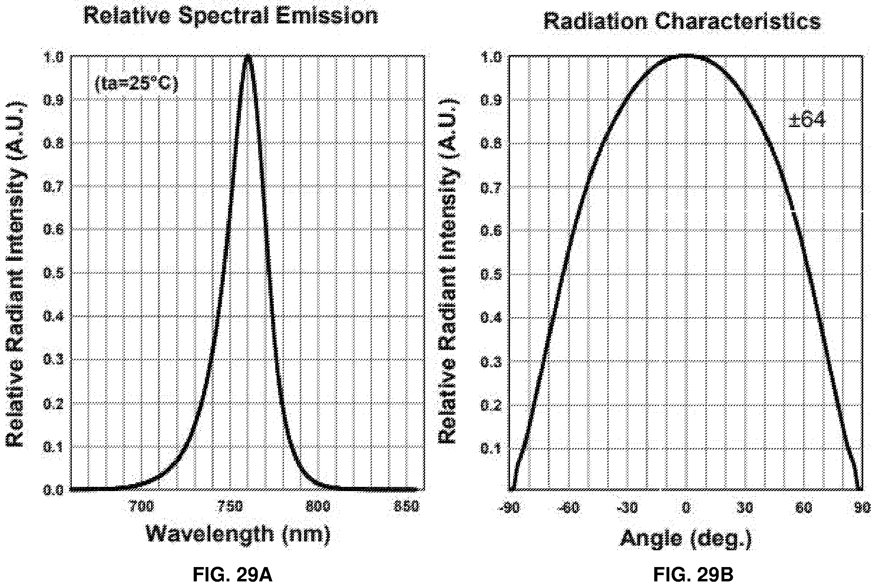

In one example embodiment, the infrared imaging endoscopic optical head 245c for use with handheld endoscopic imaging device 200 has two LED illumination sources that emit excitation light having 760 nm ± 15 nm bandwidth at FWHM for inducing fluorescence. See FIGS. 29A and 29B.

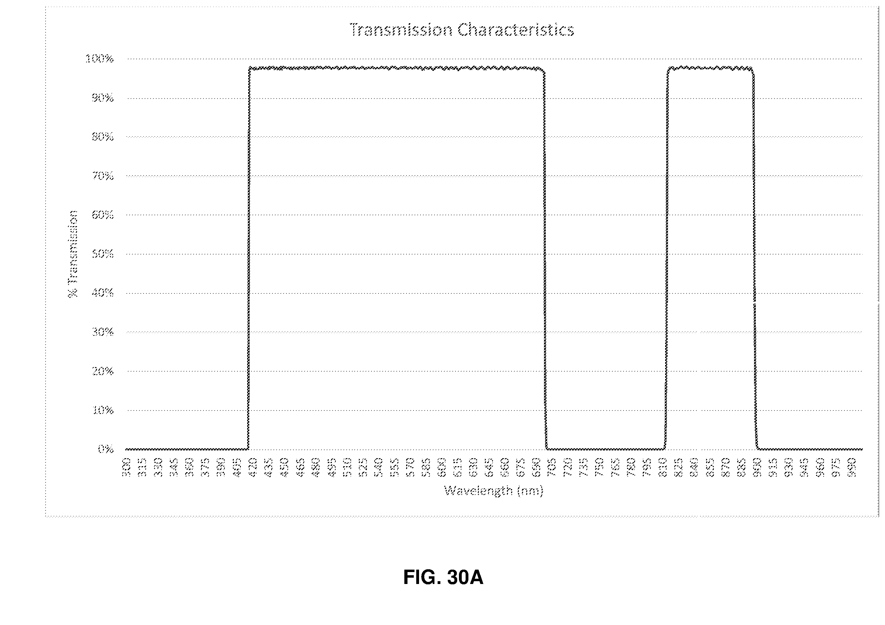

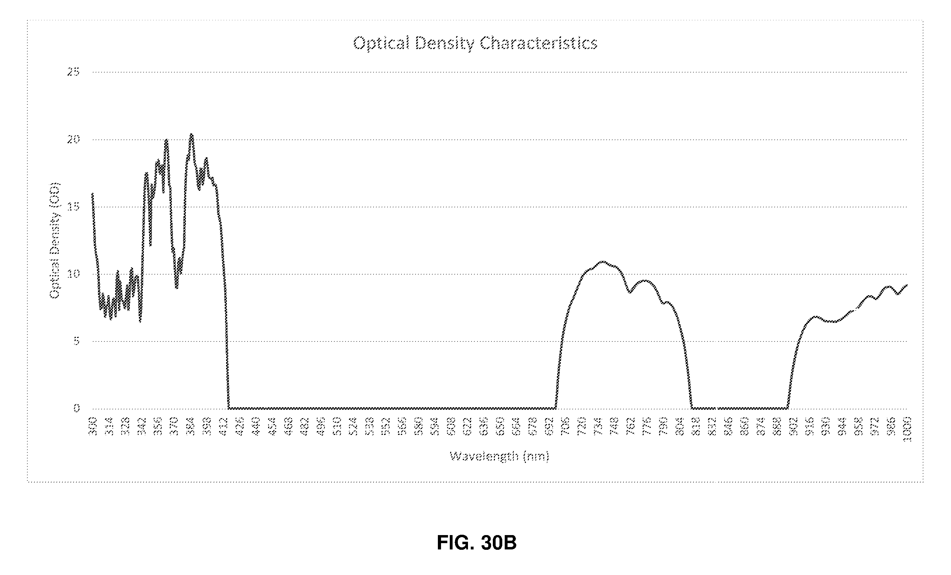

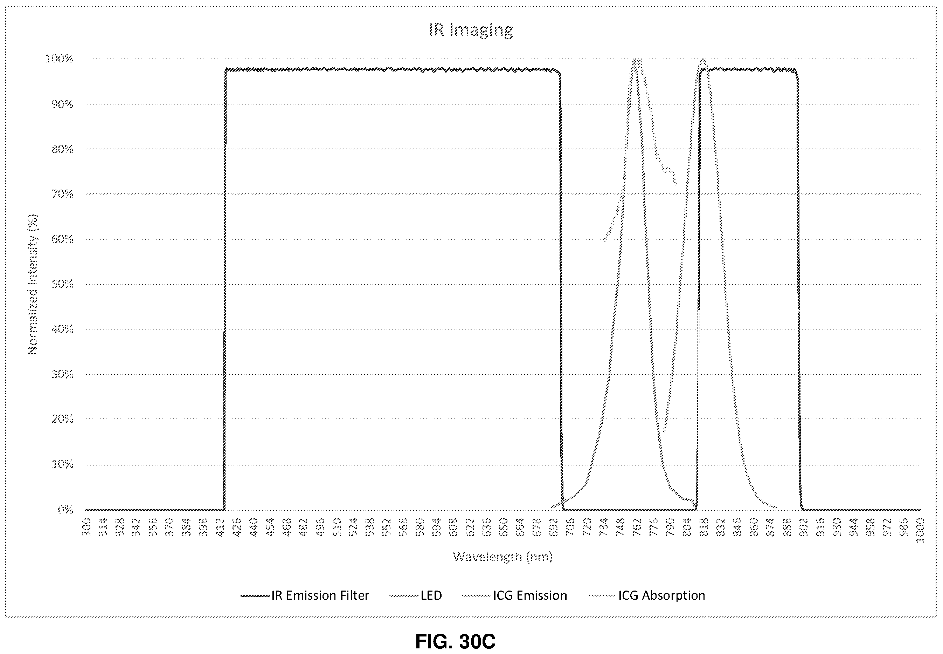

The infrared imaging endoscopic optical head 245c for use with handheld endoscopic imaging device 200 also includes a filter configured to permit passage of signals, responsive to illumination of the target with the IR excitation light, to the optical sensor (camera). In one example, the infrared imaging endoscopic optical head has a dual-bandpass emissions filter for blocking the 760 nm excitation light and capturing the resulting light emission. In one example embodiment, the dual-bandpass emission filter has the following characteristics: diameter: 6 mm (+/0.1mm), thickness: 1 mm (+/0.1mm), average transmission greater 95% for the following emission ranges: 420 nm - 700 nm and 815 nm - 895 nm. The dual-bandpass emission filter is also configured to completely block (absolute block) the following wavelengths of light: 300 nm - 412 nm, 707 nm - 804 nm, and 912 nm - 1100 nm. The characteristics of this example fixed dual-bandpass emissions filter are illustrated in FIGS. 30A, 30B, and 30C. In this embodiment, the IR passband is not centered around the peak of the emission light. This is to ensure all excitation light is blocked by the emission filter.

The infrared imaging endoscopic optical head 245c may also include additional components, such as a temperature sensor, an ambient light sensor, and/or a rangefinder.

Although discussed herein with regard to use during breast conserving surgery, the device 200 may also be used to image wounds, such as wound contained in confined spaces, for example in body canals or organs, in which a field of view might be partially or fully blocked without an endoscopic attachment. Further, although described herein as having 3 endoscopic optical heads for imaging, one for each of white light, fluorescent, and infrared imaging, it is contemplated that additional endoscopic optical heads might be provided for fluorescent imaging and/or for infrared imaging. The three endoscopic optical heads 245a, 245b, and 245c may be provided together as a kit for use with base body 210 or sold with base body 210 as a system. Additionally or alternatively, kits containing more than one fluorescent imaging endoscopic optical head 245b may be provided, wherein each fluorescent imaging endoscopic optical head 245b has an excitation light source configured to emit excitation light having a different wavelength. Such additional fluorescent imaging endoscopic optical heads 245b may also be sold individually, as supplemental components or replacement components. Similarly, kits containing more than one infrared imaging endoscopic optical head 245c may be provided, wherein each infrared imaging endoscopic optical head 245c has an excitation light source configured to emit excitation light having a different wavelength. Such additional infrared imaging endoscopic optical heads 245c may also be sold individually, as supplemental components or replacement components. Kits or systems containing a white light imaging endoscopic optical head 245a, more than one fluorescent imaging endoscopic optical head 245b and/or more than one infrared imaging endoscopic optical head 245c are also contemplated.

In another example embodiment, instead of providing three separate optical heads for white light imaging, fluorescent imaging and infrared imaging, a three-piece or three-pronged imaging endoscopic optical head may be provided, in which each of the three pieces or prongs includes a different light source for imaging. For example, a first piece or prong may include a white light source for white light imaging and an optical sensor for receiving signals responsive to illumination of a target with white light during white light imaging; a second piece or prong may include a fluorescent excitation light source for fluorescent imaging, a fluorescent emission filter to filter signals responsive to illumination of the target with the fluorescent excitation light, and an optical sensor for receiving the filtered signals responsive to illumination of the target with the fluorescent excitation light during fluorescent imaging; and a third piece or third prong may include an infrared excitation light source for infrared imaging, an infrared emission filter to filter signals responsive to illumination of the target with the infrared excitation light, and an optical sensor for receiving the filtered signals responsive to illumination of the target with the infrared excitation light during infrared imaging. Each of the fluorescent and infrared pieces (prongs) may be configured, respectively, to emit excitation light at one or more wavelengths of fluorescent and infrared excitation light. The three pieces/prongs may be joined to one another on a single mounting structure and the mounting structure may be rotatably connected to an endoscope portion of the endoscopic optical head. In this manner, the pieces/prongs may be rotated relative to the endoscope to select the desired light source of the desired mode of imaging. The rotation may be motorized or manual. Rotation of a light source into an "in-use" position may operably connect that light source to the battery pack contained in the base body to power the light source.

In an alternative embodiment, the optical sensor may be contained in the endoscope portion or the base body portion of the handheld endoscopic imaging device, with only the light sources and filters, as appropriate, included in each of the prongs. In either embodiment, the prongs may include additional components, such as temperature sensors, ambient light sensors, and/or rangefinder.

It is also contemplated that only two prongs may be provided, with the white light source and infrared light source being contained on the same prong.

In another example, instead of separate imaging heads or separate prongs, the distal tip of the endoscope portion of the handheld endoscopic imaging device may be removable and interchangeable with other distal tips, each distal tip having different characteristics such as for white light imaging, fluorescent imaging, and/or infrared imaging. As discussed above with respect to the separate endoscopic optical heads, more than one fluorescent and/or infrared imaging tip may be provided, each having a respective light source configured to emit excitation light at a predetermined wavelength. Kits of distal tips may be sold with a handheld endoscopic imaging device to create a system or sold separately as supplement or replacement components.

The number and type of excitation light sources included in an endoscopic optical housing may vary. The excitation light sources configured to emit excitation light having a wavelength of about 350 nm - about 400 nm, about 400 nm - about 450 nm, about 450 nm - about 500 nm, about 500 nm - about 550 nm, about 550 nm - about 600 nm, about 600 nm - about 650 nm, about 650 nm - about 700 nm, about 700 nm - about 750 nm, about 750 nm - about 800 nm, about 800 nm - about 850 nm, about 850 nm - about 900 nm, and/or combinations thereof. The shape of the optical housing may also vary from one housing to another, depending upon the particular application. In particular, the endoscopic portion of the optical housing may be flexible, rigid, articulatable, long, short, etc. For example, specialized shapes may be used for particular applications such as, for example, accessing confined anatomical spaces such as recesses, oral cavities, nasal cavities, ears, etc. The size of the endoscopic optical housing may vary depending upon the size and number of components contained therein. Various exemplary embodiments of the optical housings may also include, in any combination, features such as ambient light sensor, rangefinder, thermal imaging sensors, infrared radiation source to be used for three-dimensional imaging, lasers for taking measurements, etc.

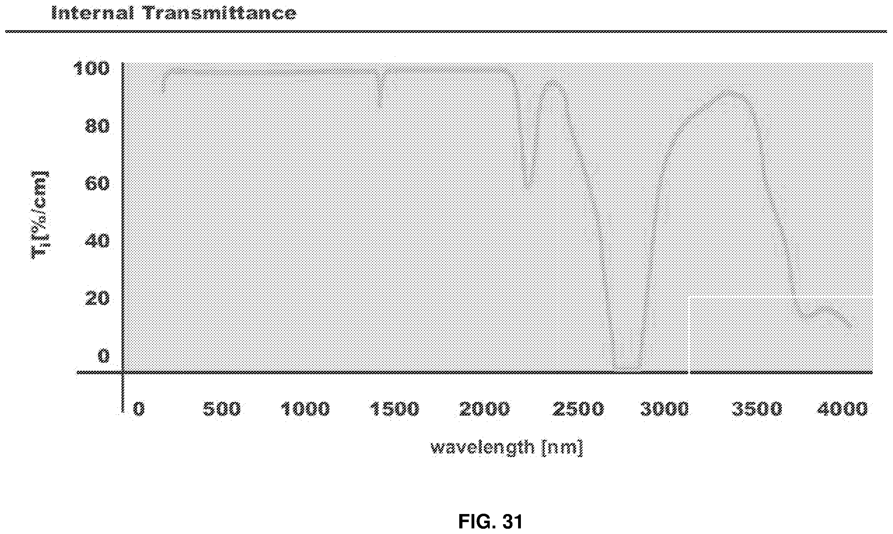

The handheld endoscopic imaging device has a protective window covering the optical components in the endoscopic head. In one example embodiment, the window may be made from Corning-Fused Silica 7980 and have a thickness of about 1.1 mm. The transmission characteristics of the window are illustrated in FIG. 31. In addition to the protective window, an optically clear lens may be provided on a custom sterile drape that is configured to be used with the device as discussed further below. All excitation light and the resulting emission light must pass though the optically clear lens of the drape.

The endoscopic portion of the endoscopic optical housing is configured to direct light onto a surgical site as tumor is being excised, a surgical margin on an outer surface of an excised tumor, on one or more sections of the excised tumor, in a surgical cavity from which the tumor/tissue has been excised, on a wound, or into a confined anatomical space. The endoscopic end may be further configured to be positioned in a surgical cavity containing a surgical margin. The base body portion of the device may comprise one or more materials that are suitable for sterilization such that the body of the device can be subject to sterilization, such as in an autoclave. An example of a suitable material is polypropylene. Those of ordinary skill in the art will be familiar with other suitable materials. Components within the body of the device that may not be capable of withstanding the conditions of an autoclave, such as electronics, may be secured or otherwise contained in a housing for protection, for example a metal or ceramic housing.

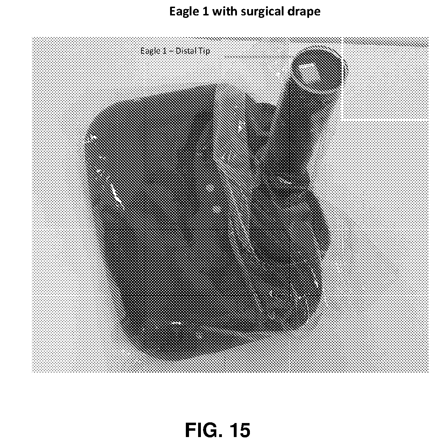

The device may be configured to be used with a surgical drape or shield. Additional details regarding such drapes can be found in PCT/CA2019/000061, filed internationally on May 9, 2019 and entitled "IMAGING DRAPES, PACKAGING FOR DRAPES, METHODS OF USE OF IMAGING DRAPES, AND METHODS FOR DEPLOYING DRAPE" and in U.S. Design Application No. 29/676,893, filed January 15, 2019 and entitled "Adaptor for Supporting a Darkening Drape". In addition to the darkening drapes disclosed in the above-referenced application, a drape for use with the imaging device of the present application may be configured to keep the surgical field sterile and/or to keep the distal tip of the endoscope from contamination with bodily fluids. These two characteristics of the drape - darkening and sterility, may be combined into a single drape or take the form of two different drape components as discussed below. An example embodiment of a drape is illustrated in FIG. 15.

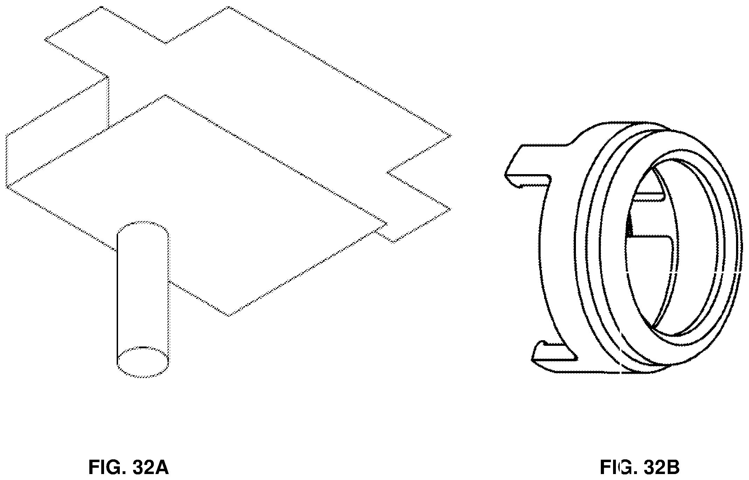

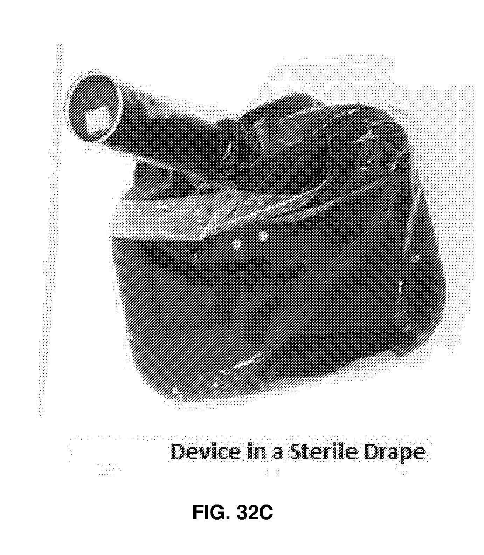

In one embodiment, shown in FIGS. 32A-32C, a custom sterile drape fits over the endoscopic head and encloses the entire handheld endoscopic imaging device. See FIGS. 32A and 32C. The sterile drape will maintain device sterility while in use. In one example embodiment, a custom sterile drape includes a lens found on the distal tip of the custom sterile drape. The lens may be optically clear and made, for example, of Sabic 9440 color 112 (clear), having a thickness of 1.8 mm and transmission at an AOI 0°: 92% and transmission AOI 45°: 88%. The custom sterile drape may have a lens holder to securely attach the drape to the distal tip of the endoscope portion of the handheld endoscopic imaging device. Once attached, the only way to remove the lens holder and drape is to break the holder. An example embodiment of the lens holder is illustrated in FIG. 32B. The optical housings may be configured such that a single adapter will fit all endoscopic optical housings to attach a darkening drape. Alternatively, a separate adaptor may be provided to engage each type of endoscopic optical housing.

For example, the inventors have found that image quality improves when ambient and artificial light are reduced in the area of imaging. This may be achieved by reducing or eliminating the ambient and/or artificial light sources in use. Alternatively, a drape or shield may be used to block at least a portion of ambient and/or artificial light from the surgical site where imaging is occurring. In one exemplary embodiment, the shield may be configured to fit over the endoscopic end of the device and be moved on the device toward and away from the surgical cavity to vary the amount of ambient and/or artificial light that can enter the surgical cavity. The shield may be cone or umbrella shaped. Alternatively, the device itself may be enclosed in a drape, with a clear sheath portion covering the end of the device configured to illuminate the surgical site with excitation light. The surgical drape may include an optically clear or transparent window that fits onto the distal tip 142 of the endoscope 140 so as to not interfere with imaging. The drape may provide protection for the handheld device to enable the handheld device to be sterilized, such as by autoclave or other sterilization methods. The drape may cover the handheld device and also provide a darkening shield that extends from the distal end and covers the area adjacent the surgical cavity to protect the surgical cavity area from light infiltration from sources of light other than the handheld device. The drape can also include or be coupled with a hard optical window, such as lens cap, that covers the distal end of the handheld device to ensure accurate transmission of light emitted from the light sources and corresponding transmission of light back to the imaging device. The body of the drape can comprise a polymer material, such as polyethylene, polyurethane, or other polymer materials. Optionally, the lens cap can comprise a different material, such as polymethyl methacrylate (PMMA) or other rigid, optically transparent polymers, glass, or other materials.

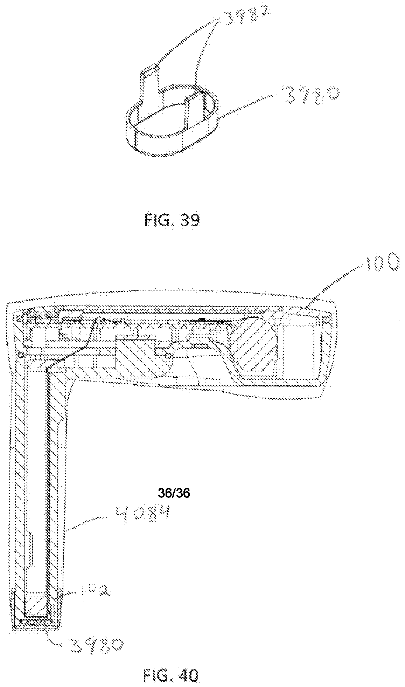

Another exemplary embodiment of a lens for a sterile drape is shown in FIG. 39. A lens 3980 comprises a shape configured to couple with the distal tip 142 of the endoscope 140. The lens 3980 can comprise a material chosen to minimize (e.g., eliminate) optical artifacts in images generated by the endoscope when the lens is positioned over the distal tip 142. The lens 3980 can also include features configured to couple the lens 3980 to the distal tip 142. For example, the lens 3980 can include one or more snap-fit arms 3982 that couple the lens 3980 to the distal tip 142 of the endoscope 140.

The lens 3980 can comprise an optically transparent material, such as for example, Sabic 9440 color 112 (clear), or other optically transparent polymer or glass materials. The lens 3980 can be coupled to a sterile drape that covers the handheld device and forms a sterile barrier between the handheld device and the surgical field. For example, referring now to FIG. 40, a cross-sectional view of a handheld device 100 with a lens 3980 in place over the distal tip 142 and a sterile drape 4084 surrounding the handheld device 100. The sterile drape 4084 can comprise flexible polymer sheet material and can be bonded to the lens 3980 by, e.g., adhesive, heat-based bonding such as laser welding, a mechanical bond, or any other type of bond sufficient to couple the lens 3980 to the sterile drape 4084.

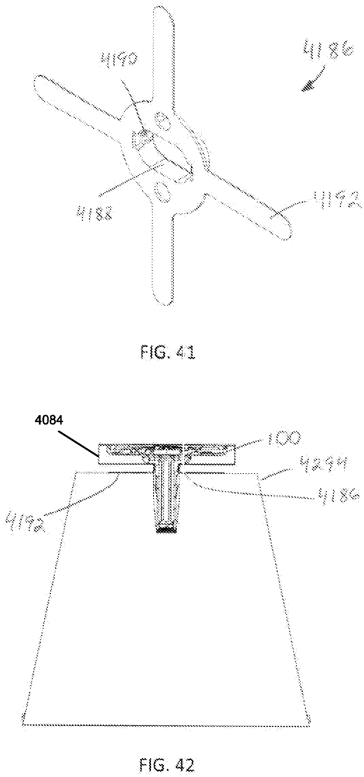

As noted above, the handheld device can be used with darkening drapes configured to limit the ambient light entering the surgical field. Such drapes can comprise multiple components, such as a darkening drape adaptor portion that couples the drape to the handheld device, and a drape portion that comprises the drape material. For example, FIG. 41 provides an example embodiment of a darkening drape adaptor portion 4186. The adaptor portion 4186 comprises an aperture 4188 configured to receive the endoscope 140 of a handheld device and coupling features 4190 in the aperture 4188 configured to engage with the endoscope 140 and retain the adaptor portion 4186 on the endoscope 140. The coupling features 4190 can comprise snap-fit features or other engagement features configured to retain the adaptor portion 4186 on the endoscope 140.

The adaptor portion 4186 further includes one or more arms 4192 configured to spread the darkening drape material out around the surgical field such that the endoscope 140 can be positioned and moved within the surgical field without the drape material interfering with the endoscope 140. For example, with reference now to FIG. 42, the adaptor portion 4186 is shown installed on the handheld device 100. The arms 4192 of the adaptor portion 4186 spread the drape material 4294 out around the endoscope 140 such that the drape material 4294 does not interfere with use of the endoscope 140 within the area surrounded by the darkening drape material 4294.

In FIG. 42, the sterile drape 4084 is also shown installed on the device 100. The sterile drape 4084 and associated lens 3980 can be installed over the device 100 as discussed above, and the endoscope portion 140, covered by the lens 3980 and sterile drape 4084, can be inserted through the aperture 4188 of the adaptor portion 4186 to install the darkening drape over the device 100. The device 100 is thereby covered by the sterile drape and the darkening drape reduces ambient light intrusion into surgical field.

Other variations on a drape configured to reduce or remove ambient and/or artificial light may be used as will be understood by those of ordinary skill in the art. Additionally or alternatively, the handheld fluorescence-based imaging device may include a sensor configured to identify if lighting conditions are satisfactory for imaging (e.g., ambient light sensor 185). The device may also be used with a surgical drape to maintain sterility of the surgical field and/or to protect the tip of the device from body fluids. The surgical drape and ambient-light reducing drape may be combined into a single drape design. Alternatively, the surgical drape may envelope the device and the ambient-light reducing drape or shield may be positioned over the surgical drape.

The endoscopic optical housing contains a number of components at the tip of the device which facilitate white light, tissue autofluorescence, PpIX fluorescence, and infrared imaging. These components include white LED(s), 405 nm LED(s), infrared LED(s) (between 750 and 800 nm), heat sink(s), at least one camera module consisting of an image sensor and lens assembly, at least one imaging filter to facilitate fluorescence imaging, ambient light sensor, a temperature sensor, and range finder. The temperature sensor is used to ensure the device is operating within a safe temperature range. The temperature sensor is used to provide feedback to a control loop which modulates the LED setup current to compensate for temperature induced efficiency change in the LED. LED radiant flux at constant drive current changes with LED temperature.

This base body portion 110 of the device has a built-in display but may also be capable of wireless image/data transmission to an external display. It is also capable of automatic digital data storage. It is also possible that the optical components of the endoscopic attachment are housed close to the base body (i.e., not at the distal tip), and incorporate fiber optics to provide imaging capabilities. In such an embodiment, the endoscope may or may not be permanently attached to the base body.

As discussed above, the endoscopic optical housing includes at least one excitation light source configured to excite autofluorescence emissions of tissue cells and fluorescence emissions of induced porphyrins in tissue cells of the surgical margin. The at least one excitation light source may be positioned on, around, and/or adjacent to the distal end of the device. Each light source may include, for example, one or more LEDs configured to emit light at the selected wavelength.

The excitation light source may provide a single wavelength of excitation light, chosen to excite tissue autofluorescence emissions and as well as fluorescence emissions of induced porphyrins in tumor/cancer cells contained in a surgical margin of the excised tumor/tissue and/or in a surgical margin of a surgical bed from which tumor/tissue cells have been excised. In one example, the excitation light may have wavelengths in the range of about 350 nm - about 600 nm, or 350 nm - about 450 nm and 550 nm - about 600 nm, or, for example 405 nm, or for example 572 nm.

Alternatively, the excitation light source may be configured to provide two or more wavelengths of excitation light. The wavelengths of the excitation light may be chosen for different purposes, as will be understood by those of skill in the art. For example, by varying the wavelength of the excitation light, it is possible to vary the depth to which the excitation light penetrates the surgical bed. As depth of penetration increases with a corresponding increase in wavelength, it is possible to use different wavelengths of light to excite tissue below the surface of the surgical bed/surgical margin. In one example, excitation light having wavelengths in the range of 350 nm - 450 nm, for example 405 nm, and excitation light having wavelengths in the range of 550 nm to 600 nm, for example 572 nm, may penetrate the tissue forming the surgical bed/surgical margin to different depths, for example, about 500µm - about 1 mm and about 2.5 mm, respectively. This will allow the user of the device, for example a surgeon or a pathologist, to visualize tumor/cancer cells at the surface of the surgical bed/surgical margin and the subsurface of the surgical bed/surgical margin. Additionally or alternatively, an excitation light having a wavelength in the near infrared/infrared range may be used, for example, excitation light having a wavelength of between about 750 nm and about 800 nm, for example 760 nm or 780 nm, may be used. In addition, to penetrating the tissue to a deeper level, use of this type of light source may be used in conjunction with a second type of imaging/contrast agent, such as infrared dye (e.g., IRDye 800, ICG). This will enable, for example, visualization of vascularization, vascular perfusion, and blood pooling within the surgical margins/surgical bed, and this information can be used by the surgeon in making a determination as to the likelihood that residual tumor/cancer cells remain in the surgical bed. In addition, the utility of visualizing vascular perfusion be to improve anastomosis during reconstruction.

The device may include additional light sources, such as a white light source for white light (WL) imaging of the surgical margin/surgical bed. In at least some instances, such as for example, during a BCS such as a lumpectomy, removal of the tumor will create a cavity which contains the surgical bed/surgical margin. WL imaging can be used to obtain an image or video of the interior of the cavity and/or the surgical margin and provide visualization of the cavity. The white light source may include one or more white light LEDs. Other sources of white light may be used, as appropriate. As will be understood by those of ordinary skill in the art, white light sources should be stable and reliable, and not produce excessive heat during prolonged use.

The base body portion 110 of the device 100 may include controls to permit switching/toggling between white light imaging and fluorescence imaging. The controls may also enable use of various excitation light sources together or separately, in various combinations, and/or sequentially. The controls may cycle through a variety of different light source combinations, may sequentially control the light sources, may strobe the light sources or otherwise control timing and duration of light source use. The controls may be automatic, manual, or a combination thereof, as will be understood by those of ordinary skill in the art. As discussed above, the touchscreen display 120 of base body portion 110 may function as a user interface to allow control of the device 100. Alternatively, it is contemplated that separate controls, such as hand-actuated buttons, may be used instead of or in addition to touchscreen controls. Such hand-actuated buttons may be positioned, for example, on the handgrips to allow the user to easily actuate the controls while holding and using the device.

The endoscopic optical housing portion 145 of the device 100 contains one or more optical imaging filters configured to prevent passage of reflected excitation light and permit passage of emissions having wavelengths corresponding to autofluorescence emissions of tissue cells and fluorescence emissions of the induced porphyrins in tissue cells. In one example embodiment, the device includes one filter for white light (WL) imaging and infrared (IR) imaging, and another filter for fluorescence (FL) imaging. The device may be configured to switch between different imaging filters based on desired imaging mode and the excitation light emitted by the handheld device.

The endoscopic optical housing may be modified by using optical or variably oriented polarization filters (e.g., linear or circular combined with the use of optical wave plates) attached in a reasonable manner to the excitation/illumination light sources and the imaging sensor. In this way, the device may be used to image the tissue surface with polarized light illumination and non-polarized light detection or vice versa, or polarized light illumination and polarized light detection, with either white light reflectance and/or fluorescence imaging. This may permit imaging of tissues with minimized specular reflections (e.g., glare from white light imaging), as well as enable imaging of fluorescence polarization and/or anisotropy-dependent changes in connective tissues (e.g., collagens and elastin) within the tissues. The ability to use polarization optics in the endoscopic optical housing enables either polarization of reflected light or fluorescence light from a target. This may potentially provide improved image contrast where tumor vs normal tissues reflect 405 nm excitation light differently or emit different polarization information from the 500-550 nm and 600-660 nm emitted fluorescence light.

The handheld fluorescence-based imaging device also includes an imaging lens and an image sensor in the endoscopic optical housing of the device. The imaging lens or lens assembly may be configured to focus the filtered autofluorescence emissions and fluorescence emissions on the image sensor. A wide-angle imaging lens or a fish-eye imaging lens are examples of suitable lenses. A wide-angle lens may provide a view of 180 degrees. The lens may also provide optical magnification. A very high resolution is desirable for the imaging device, such that it is possible to make distinctions between very small groups of cells. This is desirable to achieve the goal of maximizing the amount of healthy tissue retained during surgery while maximizing the potential for removing substantially all residual cancer cells, precancer cells, satellite lesions. The image sensor is configured to detect the filtered autofluorescence emissions of tissue cells and fluorescence emissions of the induced porphyrins in tissue cells of the surgical margin. The image sensor may have 4K video capability as well as autofocus and optical or digital zoom capabilities. CCD or CMOS imaging sensors may be used. In one example, a CMOS sensor combined with a filter may be used, i.e., a hyperspectral image sensor, such as those sold by Ximea Company. Example filters include a visible light filter (https://www.ximea.com/en/products/hyperspectral-cameras-based-on-usb3-xispec/mq022hg-im-sm4x4-vis) and an IR filter (https://www.ximea.com/en/products/hyperspectral-cameras-based-on-usb3-xispec/mq022hg-im-sm5x5-nir). The handheld device also may contain a processor configured to receive the detected emissions and to output data regarding the detected filtered autofluorescence emissions of tissue cells and fluorescence emissions of the induced porphyrins in tissue cells of the surgical margin. The processor may have the ability to run simultaneous programs seamlessly (including but not limited to, wireless signal monitoring, battery monitoring and control, temperature monitoring, image acceptance/compression, and button press monitoring). The processor interfaces with internal storage, buttons, optics, and the wireless module. The processor also has the ability to read analog signals.

The device may also include a wireless module and be configured for completely wireless operation. It may utilize a high throughput wireless signal and have the ability to transmit high definition video with minimal latency. The device may be both Wi-Fi and Bluetooth enabled - Wi-Fi for data transmission, Bluetooth for quick connection. The device may utilize a 5 GHz wireless transmission band operation for isolation from other devices. Further, the device may be capable of running as soft access point, which eliminates the need for a connection to the internet and keeps the device and module connected in isolation from other devices which is relevant to patient data security. The device may be configured for wireless charging and include inductive charging coils. Additionally or alternatively, the device may include a port configured to receive a charging connection.

In accordance with one aspect of the present disclosure, the endoscopic handheld device may be used to obtain three-dimensional fluorescent images of the target. Systems for and methods of obtaining such three-dimensional images are disclosed in U.S. Provisional Application No. 62/793,837 filed January 17, 2019 and entitled "Systems Methods, and Devices for Three-Dimensional Imaging, Measurement, and Display of Wounds and Tissue Specimens".

Additional details regarding the construction, functionality, and operation of exemplary devices described here can be found in U.S. Provisional Applications 62/625,983 (filed Feb. 3, 2018) titled "Devices, Systems, and Methods for Tumor Visualization and Removal" and 62/625,967 (filed Feb. 02, 2018) titled "Devices, Systems, and Methods for Tumor Visualization and Removal".

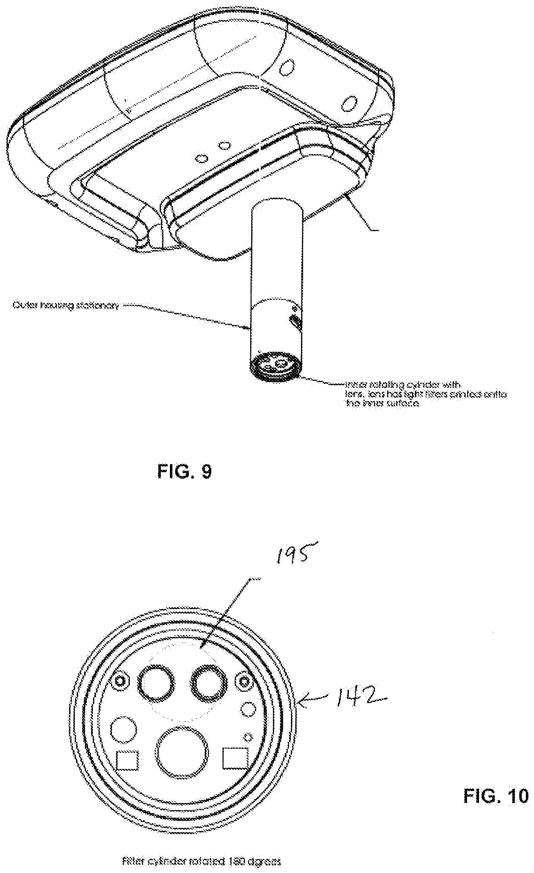

As embodied in one example (see FIGS. 9-12), a distal end of the endoscope includes one or more light sources, such as light-emitting diodes (LEDs) configured to emit a having a specific wavelength. For example, the one or more light sources can be configured to emit wavelengths of 405nm, 760nm, 780nm, or other wavelengths. The distal end further includes an imaging device, such as a camera assembly configured to capture images of the surgical cavity illuminated by the one or more light sources. The distal end further includes one or more spectral filters positioned to filter the light entering the imaging device, as discussed in greater detail below.

In some exemplary embodiments, the handheld device can include a channel formed in a lateral wall of the distal end. The channel can be used to facilitate insertion of additional tools, such as optical fibers for an auxiliary light source or auxiliary imaging sensor, a cauterizing tool, biopsy forceps, a tagging tool (for marking tissue with a clip, optical tag, dye or paint, etc.), or other tools, while the handheld device 100 is in position within a surgical site. Alternatively or additionally, some embodiments could include a channel formed within the distal tip of the device, i.e., an internal channel within the device for introduction of any of the above-mentioned tools into the surgical site while the handheld device is in use.

As discussed in greater detail below, the handheld device includes various electrical subsystems including one or more imaging devices, such as one or more camera sensors, one or more fluorescent light LEDs, one of more infrared LEDs, one or more white light LEDs, and various sensors such as temperature sensors, ambient light sensors, and range finding sensors. In some exemplary embodiments, the handheld device includes two camera sensors, each configured to support image capture based on a differing range of wavelengths. Other components can include one or more of LED drivers that generate drive voltages to drive the LEDs as required to achieve the setpoint drive current, one or more accelerometers and gyroscopes to allow a video stream to be tagged with the position of the handheld device, e.g., to provide spatial orientation of features within the surgical cavity, flash memory to provide local storage of videos and still images, a USB hub to provide an interface for factory load of software, test, and calibration of the handheld device, an inductive battery charging system, motor drive electronics to provide automatic switching of optical filters as discussed below, a Wi-Fi radio subsystem, a user interface providing information regarding to mode the device to the user, a rechargeable battery (such as a Li-lon battery) an audio device such as a speaker for providing audible feedback of the system state to the user, an audio recording device, and other components. Such components can be operatively coupled with one or more controllers, such as computer processors, housed within the handheld device.

For example, in an embodiment, the handheld device includes one or both of an application processer and a microcontroller unit. The application processor can perform functions including, but not limited to, sending the camera interface and video stream (e.g., still images and motion video) to the wireless transmission function to transmit the data to a display or computer terminal, interfacing with the accelerometer, gyroscope, and on-board flash memory, interfacing with the microcontroller unit, driving the speaker for audible feedback to the user, and managing the wireless communications subsystem.

The microcontroller unit can provide functions such as control the LED drive electronics including the temperature compensation loops, communication with the temperature sensor, the ambient light sensor, and the range finder, and interfacing with the application processor for conveying and receiving system usage and context state. The microcontroller unit can also monitor the system for exception conditions, control indicator LEDs, monitor pushbuttons or other user interface devices, control the motor drive for switching between optical filters, monitor the wireless battery charging and charge state and control power management, as well as other functions.

The handheld device can include one or more printed circuit board (PCB) components to facilitate manufacture and assembly of the handheld device. The handheld device includes an LED PCB that can include one or more light emitting diodes (LEDs) and associated electrical components. The LED PCB can be operatively coupled with other electronic systems in the handheld device through wiring (such as a bus), and can be connected to control systems of the handheld device such as controls for a power source such as a battery, etc.

A distal PCB can be positioned adjacent the imaging device and can include components supporting the imaging device, such as components that interface the imaging device with the controls and a power supply, e.g., battery, of the handheld device. In some embodiments, the light sources of the handheld device can be included on the distal PCB.

For example, an exemplary layout for a distal end PCB may include first and second LED devices. As a non-limiting example, the first and second LED devices can comprise an LED configured to emit light having a 405 nm wavelength, while the second LED device can comprise an LED configured to emit light with a 760 nm wavelength, a 780 nm wavelength, or other wavelengths. The PCB can further include a white light LED configured to provide visual illumination to the area to be imaged.

The distal PCB can include other components operatively coupled with a control system of the handheld device and configured to provide other information to the control system to support effective operation of the handheld device. For example, the distal PCB can include a temperature sensor used to provide feedback to an LED setpoint temperature compensation loop to ensure the system is operating within a safe temperature range. In addition to ensuring the system is operating within a safe temperature range, the temperature sensor provides feedback to an LED setpoint temperature compensation loop to minimize the change in LED radiant flux as temperature changes. A range finder can measure the distance between the camera sensor and the target being imaged and can be used to provide feedback to the user to guide the user on imaging at the correct distance. A change in measured target distance can optionally be used to initiate a camera sensor refocus action. An ambient light sensor can provide feedback to a user regarding the level of ambient light, as the fluorescence imaging is only effective in an adequately dark environment. The measured ambient light level could also be useful during white light imaging mode to enable the white light LED or control its intensity. The distal PCB can be operatively coupled with other portions of the handheld device, such as the controls, a power supply such as a battery, one or more processors, such as the microcontroller unit and the application processor, or other components.

The LED devices can be controlled by a closed-loop system using information from the temperature sensor as input to a control loop which adjusts the LED drive current setpoint. In some embodiments, low and high range LED intensity modes may be supported for different applications. Examples include imaging at close range within a surgical cavity and lumpectomy imaging in the pathology suite at far range.

FIG. 36 provides an example layout of hardware components of devices according to various embodiments of the disclosure. Referring to FIG. 36, hardware components of a handheld device according to an exemplary embodiment of the disclosure are grouped in an optical printed circuit board (PCB) 3600 (which may correspond to the distal end PCB discussed herein) and an electronics system 3602. The optical PCB 3600 includes fluorescent LEDs 3604, infrared LEDs 3606, and white light LEDs 3608. The optical PCB 3600 can also include an ambient light sensor 3610, a range finder such as laser range finder 3612, and a temperature sensor 3614.

The optical PCB 3600 is operably coupled to the electronics system 3602. The electronics system can include electronic control components such as an application processor module 3616, a real time microcontroller unit (MCU) 3618, and a power management subsystem 3620. The electronics system 3602 can also include components and systems that interface with other electronic components of the handheld imaging device. For example, the electronics system 3602 can include a CMOS camera interface 3622 and LED drivers 3624 for the fluorescent, infrared, and white light LEDs.

Other supporting electronic systems and components of the electronics system 3602 can include memory, such as a flash memory device 3626, spatial and motion sensors 3628 such as one or more of a magnetometer, accelerometer, and gyroscope, a Wi-Fi radio subsystem 3630, and a USB hub 3632. Contacts 3310 are configured to touch contacts 3308 (FIG. 34) of the docking station 3302 when the handheld device 3300 is placed on the docking station 3302, as discussed above in connection with FIG. 34.

The electronics system 3602 can include various connections and/or associated connectors to facilitate coupling of the electronics system 3602 to other components of the handheld device. For example, the electronics system 3602 can include one or more of a fluorescent camera connector 3634 configured to operably couple a fluorescent camera 3635 with the electronics system 3602, white light/infrared camera connector 3636 configured to operably couple a white light/infrared camera 3637 with the electronics system 3602, a display connector 3638, a speaker connector 3640, a Wi-Fi antenna connector 3642, and a battery pack connector 3644. The various connectors can provide an electrical coupling from the electronics system 3602 to the respective components of the handheld device, e.g., the various camera devices, a display, a speaker or other auditory device, a Wi-Fi antenna, and a battery pack as discussed herein.

The electronics system 3602 can include various user controls and related indicators. For example, the electronics system 3602 can include user controls such as a power switch 3646 and associated indicator 3648, a charging status indicator 3650, picture capture switch 3652, video capture switch 3654, and fluorescent, white light, and infrared imaging mode switches 3656, 3658, and 3660 respectively.

The electronics system can further include one or more connectors to facilitate coupling of the handheld device to a computer, such as, for example, a universal serial bus (USB) connector. In the exemplary embodiment of FIG. 36, the connector is a USB type-C (USB-C) connector 3662. The USB connection provided by the USB connector can provide an alternative to wireless transmission of data over Wi-Fi or other wireless protocols, such as Bluetooth, which can be necessary due to restrictions on wireless devices in some operating rooms. Further, the USB connection can be used for software and/or firmware updates, battery charging, and other functions.

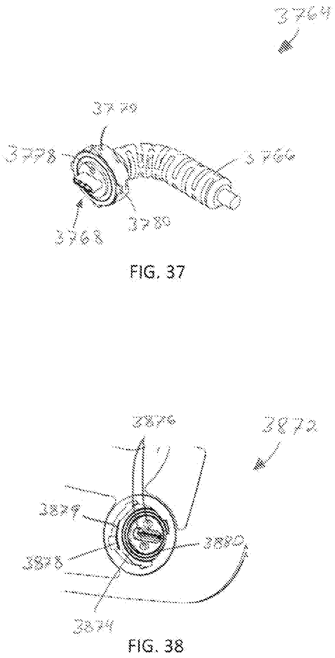

The electronics system 3602 can be operatively coupled to a computer by a removable USB connection cable, such as cable 3764 shown in FIG. 37. The cable 3764 can include various features configured to ensure that the cable does not interfere with the surgical field and to ensure the cable is not inadvertently removed from the handheld device during use. While the description herein may refer to universal serial bus (USB) type connections, it should be understood that the present disclosure is not limited to any specific connection protocol, and connection protocols other than the various types of USB interfaces are within the scope of the disclosure.

The cable 3764 can include a strain relief feature 3766 molded to facilitate keeping the cable from interfering with the surgical field. For example, in the embodiment of FIG. 37, the cable 3764 is configured to be inserted into a connection port on the back of a handheld device according to the present disclosure. The strain relief 3766 is molded to create a general 90-degree curvature in the cable 3764 when in an unstressed state. The curvature of the cable 3764 facilitates routing of the cable 3764 away from the surgical field. The curvature of the cable may be less than or greater than 90 degrees. As an exemplary range, the curvature of the cable can be, without limitation, from 70 degrees to 110 degrees. Curvatures of less than 70 degrees or greater than 110 degrees are within the scope of the disclosure. The particular shape of the cable imparted by the strain relief feature 3766 can depend on the location of the connection port on the handheld device. For example, for a handheld device with a connection port on the side, the strain relief could be straight to route the cable away from the surgical field.

The cable 3764 can also include a connection interface 3768 configured to electrically and mechanically couple the cable 3764 to the handheld device. The connection interface 3768 can include a locking ring 3770 that provides a positive mechanical engagement between the cable 3764 and the handheld device to prevent the cable 3764 from being inadvertently pulled from the handheld device during use.

For example, referring now to FIG. 38, a portion of a housing 3872 is shown, including a connection port 3874 configured to receive the connection interface 3768 of the cable 3764. The connection port 3874 includes a surrounding portion including slots 3878 configured to receive corresponding tabs 3780 of the locking ring 3770. After the locking ring 3770 is inserted such that the tabs 3780 of the locking ring 3770 are received in the slots 3878, the locking ring 3770 is rotated such that the tabs 3780 rotate into circumferentially extending portions 3879 of the slots 3878, and the locking ring 3770 retains the connection interface 3768 within the connection port 3874.

The locking ring 3770 and the surrounding portion can comprise materials having sufficient mechanical strength to withstand forces that may be applied to the connection interface 3768 in use. For example, one or both of the locking ring 3770 and the surrounding portion of the connection port 3874 can comprise a metal such as an aluminum alloy, a high strength polymer, composite material, or other material.

Because the strain relief feature 3766 routes the cable away from the handheld device, application of force to the cable 3764 and/or strain relief feature 3766 can create a relatively large torque at the connection interface 3768 due to the strain relief feature 3766 acting as a moment arm. The connection interface 3768 of the cable 3764 and a corresponding connection port on the housing of the handheld device can include features configured to withstand such torque and other forces without applying these forces to the more sensitive electrical contact components of the connection interface 3768 and corresponding connection port.

For example, the connection port 3874 can include pins 3876 extending from a face of the port 3874. The connection interface 3768 of the cable 3764 include recesses 3778 (only one of which is shown in FIG. 37) into which the pins 3876 are received. The pins 3876 and recesses 3778 form a mechanical interface between the connection port 3874 and connection interface 3768 that has mechanical strength sufficient to withstand typical forces to which the cable 3764 and connection port 3874 are subjected during use and prevents undue stress from being placed on the electrical interface components of the connection port 3874 and connection interface 3768.

Additionally, in some exemplary embodiments, one or both of the connection port 3874 and the connection interface 3768 can include a seal to prevent intrusion of various contaminants such as biological or therapeutic liquids or substances into the electrical contacts of the connection port 3874 and connection interface 3768. For example, in the embodiment of FIG. 38, the connection port 3874 includes a gasket 3880 that forms a seal against the connection interface 3768 when the connection interface 3768 is secured in the connection port 3874 with the locking ring 3770. Additionally, in some embodiments, the gasket or other seal can be configured to provide a preload force between the connection port 3874 and connection interface 3768 that serve to keep the locking ring 3770 secure in a coupled state of the connection interface 3768 in the connection port 3874. When in the coupled state, the cable 3764 can provide a data and/or power transmission conduit for the handheld device to be attached to a computer, as discussed above. Further, the cable 3764 can be provided with a sterile sheath configured to attach to the sterile drape 4084 (FIG. 40) to maintain a sterile barrier between the handheld device 100 and the surgical field when the cable 3764 is coupled to the connection port 3874.

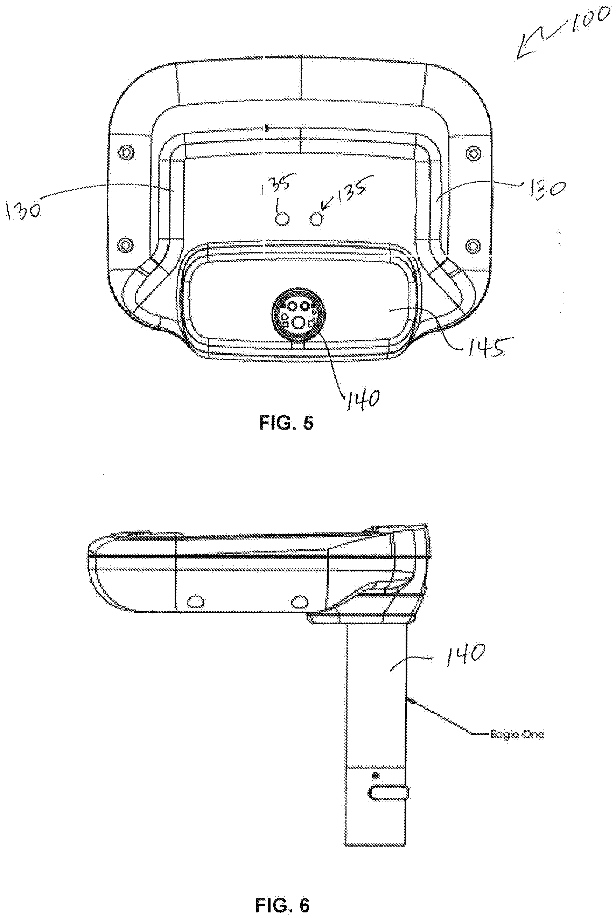

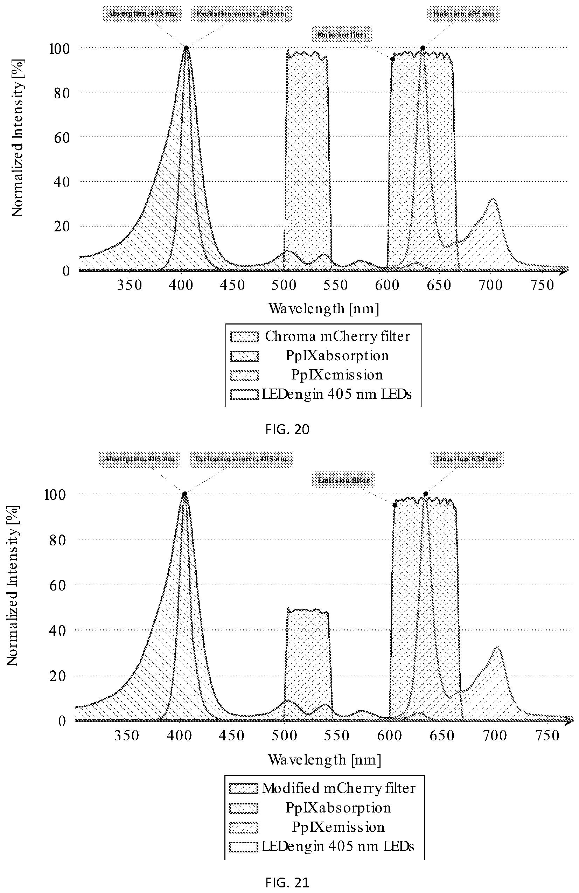

As noted above, the handheld device can include one or more optical filters configured to permit passage of a specific light wavelength or wavelength band while blocking other wavelengths. By positioning such a filter between the imaging device 520 (FIG. 6) and the area to be imaged, a particular wavelength or band of wavelengths is isolated in the image and permits visualization of the areas emitting light in that wavelength or wavelength band. For example, the handheld device can include one or more of a notch filter configured to pass a specified wavelength, an mCherry filter (as used herein mCherry filter may refer to a filter that transmits green (approximately 500-550 nm wavelength of emission light) and red (approximately 600-660 nm wavelength of emission light), or other types of optical spectral filters. A spectrum for the mCherry filter is shown in FIG. 20 and more information can be found at https://www.chroma.com/products/parts/59022m.

The one or more filters may be configured such that the user can switch between the one or more filters when using different light sources, different compounds or dyes, etc. Such switching of filters can be carried out automatically based on other user-defined settings of the handheld device, such as a mode chosen by the user.

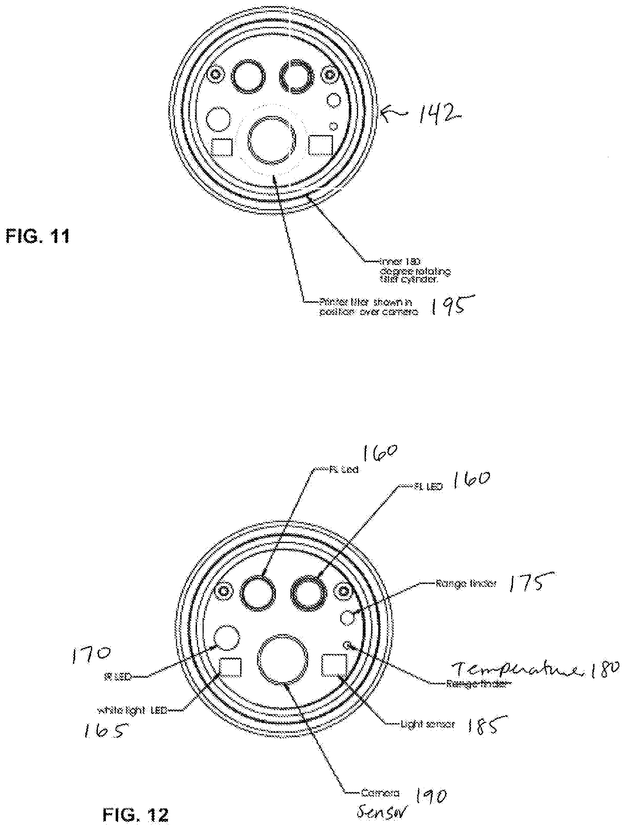

The handheld device can include components configured to enable filters to be switched quickly in a manual or automatic fashion. For example, a filter wheel in accordance with an embodiment of the present disclosure is shown. The filter wheel can be positioned on the handheld device between the imaging device (e.g., imaging device) and the area to be imaged. For example, a filter wheel can be distal of the imaging device in the distal end of the handheld device. As illustrated, for example, in FIGS. 10 and 11, the filter 195 can be rotated between a first configuration in which the filter is positioned over the fluorescent excitation light sources 160 and a second position in which the filter 195 is positioned over the camera sensor 190.

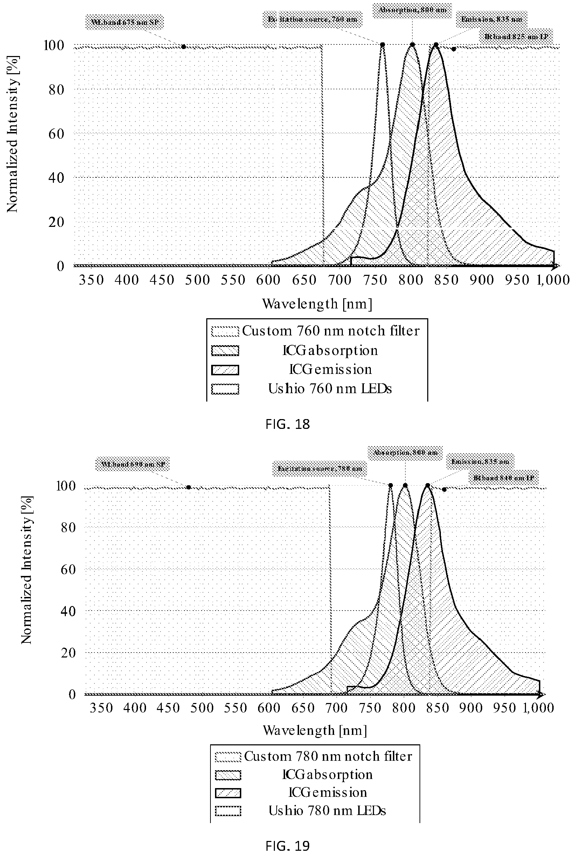

The filter wheel includes a first optical filter configured to support white light and infrared (WL/IR) imaging, and a second optical filter configured to support fluorescence (FL) imaging. The first filter and second filter are positioned opposite one another across a rotational axis A_{R} of the filter wheel about which the filter wheel is rotatable. As discussed above, the imaging device can be in an offset position such that each of the first filter and second filter can alternatingly be positioned in front of the imaging device as desired by the user. The filter wheel can be rotated manually by a user or can be automated. As discussed in greater detail below in connection with FIGS. 18-23, the user can choose one of the first filter and second filter based on the compound or dye used and/or the wavelength of the excitation light applied to the surgical cavity. Additionally or alternatively, rotation of the filter wheel can be done manually, such as by providing on the filter wheel a circumferential surface that can be gripped by a user. While the filter wheel is described as including two filters, other embodiments of filter wheels could include three filters, four filters, or any desired number of desired filters that can be fit on the filter wheel.

In an exemplary embodiment, the first filter comprises a notch filter configured to block light having a wavelength of from 675 nm to 825 nm, while allowing passage of wavelengths less than 675 nm and greater than 825 nm. In a different embodiment, the first filter can comprise a notch filter configured to block light having a wavelength of from 690 nm to 840 nm, while allowing passage of wavelengths less than 690 nm and greater than 825 nm. The second filter can comprise an mCherry filter having the characteristics discussed in connection with FIGS. 18-23 below.

FIGS. 18-23 provide examples of potential usage scenarios of the handheld device according to various embodiments of the disclosure. Referring now to FIG. 18, in this usage scenario, the tissue is illuminated with a light source (such as one or more LEDs of the handheld device) providing an excitation light wavelength of 760 nm. The tissue is treated with an IR dye such as ICG. A filter positioned to filter light entering an imaging device, such as imaging device comprises a 760 nm notch filter that filters the excitation light from being captured by the imaging sensor. The filter has a notch between 675 nm and 825 nm. As seen in the chart of FIG. 18, the light emitted from the ICG-treated tissue has an emission wavelength of 835 nm, and thus passes through the notch filter and is captured by the image sensor, thereby generating an image with revealing the ICG-treated tissue.

Referring now to FIG. 19, tissue is illuminated with a light source having a wavelength of 780 nm. A notch filter having a short pass wavelength of 690 nm and a long pass wavelength of 840 nm is used to filter light returning to the imaging device. The tissue is treated with an IR dye such as ICG, and when excited by the 780 nm light source, emits a light with a peak intensity wavelength of 835 nm and passes through the notch filter to be captured by the imaging device, again revealing the ICG-treated tissue in the resulting image.

FIGS. 20-23 are example use scenarios where the handheld device is used for fluorescence imaging to improve tumor-to-normal contrast. Referring now to FIG. 20, the subject may be given a diagnostic dose of aminolevulinic acid (ALA) to induce PpIX formation in tumor tissue. The tissue is illuminated with a light source having a wavelength of 405 nm. A Chroma mCherry filter is used to filter light captured by the imaging device. As shown in FIG. 20, the PpIX emits light with a wavelength of 635 nm, within the red transmission band of the mCherry filter, and thus can be captured by the imaging device, revealing in the resulting image the tissue in which PpIX formation was induced.

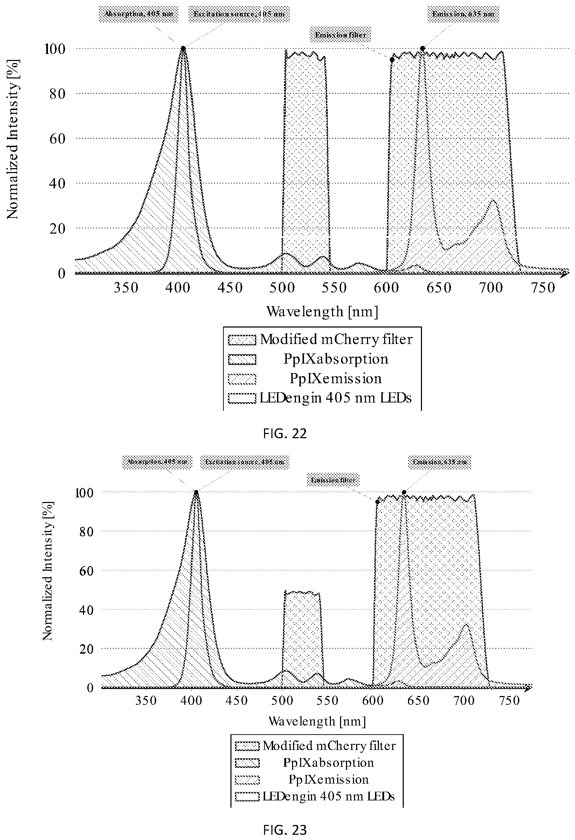

FIGS. 21-23 present examples similar to the example of FIG. 20, with various modifications made to the mCherry filter to improve tumor-to-normal contrast. In FIG. 21, the mCherry filter is modified to reduce the green band transmission. The amount of transmission may be reduced, for example, by between about 10 percent and about 60% and in the example shown may be reduced by approximately 50%. In FIG. 22, the mCherry filter is modified to widen the red transmission band to 600-725 nm (from approximately 600-675 nm shown in FIGS. 20 and 21). In FIG. 23, the mCherry filter is modified both to reduce the green band transmission by 50% and to widen the red band transmission to 725 nm.

According to embodiments of the disclosure, changing from the white light and infrared imaging modes discussed in connection with FIGS. 18 and 19 to the fluorescence imaging modes discussed in connection with FIGS. 20-23 can be accomplished by rotating the filter wheel 195 such that the desired filter (e.g., the notch filter or the mCherry filter) is positioned in front of the imaging device to filter the light wavelengths returning to the handheld device. Controls on the handheld device, such as controls can include switches, buttons, etc. to switch light sources from the 760 nm or 780 nm LEDs to the 405 nm LEDs or to white LEDs. In some embodiments of the disclosure, the filter wheel can be configured to automatically rotate from one filter to another based on a chosen mode of the handheld device, e.g., input by a user at the controls.

The handheld device can be further configured to provide imaging modes in addition to those described above. For example, the handheld device can include a mode in which the image sensor, the light source, and the filter are configured to provide 3D imaging for topographic mapping of an imaging surface. Additional details regarding the use of 3D imaging can be found in provisional application no. 62/793,837 entitled "Systems Methods, and Devices for Three-Dimensional Imaging, Measurement, and Display of Wounds and Tissue Specimens," filed January 17, 2019.

As an example of another imaging mode, the handheld device can be configured to provide fluorescence lifetime imaging of tissue. Fluorophores such as PpIX have a fluorescence emission decay profile that defines how quickly the visible fluorescence light will die out once the excitation source is removed. Thus, by capturing images shortly after excitation sources are removed or turned off, different fluorophores can be imaged in isolation by tailoring an image capture time for each unique fluorophore after the excitation source that excited the specific fluorophore is removed. For example, if PpIX and another fluorophore have decay times of 9 ns and 5 ns respectively, PpIX can be imaged in isolation by capturing an image between 5 and 9 ns after the excitation source is removed. In this manner, fluorescence lifetime imaging can enable detection of multiple unique fluorophores by their respective fluorescence lifetime signatures based on the differences in the exponential decay rate of the fluorescence from the fluorophore. Such time-resolved fluorescence imaging methods could be achieved by pulsing various excitation wavelength LEDS and gating imaging sensors to detect fluorophore lifetimes of interest. Fluorescence lifetime imaging of tissue could be used to identify and differentiate between different tissue components, including health and diseased tissues but also other biological components such as microorganisms and intrinsically-fluorescence chemical agents or drugs.

Other possible imaging modes could include various combinations of white light imaging, infrared imaging, and fluorescence imaging. For example, in one possible imaging mode, both white light and IR light sources are used to illuminate the tissue. An infrared dye, such as ICG, may be excited by the IR light source and the resulting IR imagery can be overlaid on the white light image to show the IR imagery in anatomical context.

In another imaging mode, white light illumination is followed by 405 nm light illumination. The imaging filter for WL/IR is used during white light illumination, and the FL filter is used during 405 nm illumination. Sequential images of white light and fluorescence are captured and can be overlaid to provide anatomical context (white light image) for the tumor location (FL image).

It will be appreciated by those ordinarily skilled in the art having the benefit of this disclosure that the present disclosure provides various exemplary devices, systems, and methods for intraoperative and/or *in vitro* visualization of tumors and/or residual cancer cells on surgical margins. Further modifications and alternative embodiments of various aspects of the present disclosure will be apparent to those skilled in the art in view of this description.

Furthermore, the devices and methods may include additional components or steps that were omitted from the drawings for clarity of illustration and/or operation. Accordingly, this description is to be construed as illustrative only and is for the purpose of teaching those skilled in the art the general manner of carrying out the present disclosure. It is to be understood that the various embodiments shown and described herein are to be taken as exemplary. Elements and materials, and arrangements of those elements and materials, may be substituted for those illustrated and described herein, parts and processes may be reversed, and certain features of the present disclosure may be utilized independently, all as would be apparent to one skilled in the art after having the benefit of the description herein. Changes may be made in the elements described herein without departing from the scope of the present disclosure and following claims, including their equivalents.

It is to be understood that the particular examples and embodiments set forth herein are non-limiting, and modifications to structure, dimensions, materials, and methodologies may be made without departing from the scope of the present disclosure.

Furthermore, this description's terminology is not intended to limit the present disclosure. For example, spatially relative terms-such as "beneath," "below," "lower," "above," "upper," "bottom," "right," "left," "proximal," "distal," "front," and the like-may be used to describe one element's or feature's relationship to another element or feature as illustrated in the figures. These spatially relative terms are intended to encompass different positions (i.e., locations) and orientations (i.e., rotational placements) of a device in use or operation in addition to the position and orientation shown in the drawings.

For the purposes of this specification and appended claims, unless otherwise indicated, all numbers expressing quantities, percentages or proportions, and other numerical values used in the specification and claims, are to be understood as being modified in all instances by the term "about" if they are not already. Accordingly, unless indicated to the contrary, the numerical parameters set forth in the following specification and attached claims are approximations that may vary depending upon the desired properties sought to be obtained by the present disclosure. At the very least, and not as an attempt to limit the application of the doctrine of equivalents to the scope of the claims, each numerical parameter should at least be construed in light of the number of reported significant digits and by applying ordinary rounding techniques.

Notwithstanding that the numerical ranges and parameters setting forth the broad scope of the present disclosure are approximations, the numerical values set forth in the specific examples are reported as precisely as possible. Any numerical value, however, inherently contains certain errors necessarily resulting from the standard deviation found in their respective testing measurements. Moreover, all ranges disclosed herein are to be understood to encompass any and all sub-ranges subsumed therein.

It is noted that, as used in this specification and the appended claims, the singular forms "a," "an," and "the," and any singular use of any word, include plural referents unless expressly and unequivocally limited to one referent. As used herein, the term "include" and its grammatical variants are intended to be non-limiting, such that recitation of items in a list is not to the exclusion of other like items that can be substituted or added to the listed items.

It should be understood that while the present disclosure has been described in detail with respect to various exemplary embodiments thereof, it should not be considered limited to such, as numerous modifications are possible without departing from the broad scope of the appended claims, including the equivalents they encompass.

## Claims

1. A portable, handheld endoscopic imaging system (100), comprising: an endoscope (140);
a base body portion (110), with a front or user-facing side (115) of the base body portion (110) including a display (120) for displaying images and videos captured by the system (100); wherein the endoscope (140) is positioned on an opposite, patient-facing side (125), of the system (100);
at least one excitation light source (160, 170) located at a distal tip (142) of the endoscope (140) and configured to excite autofluorescence emissions of tissue cells and fluorescence emissions of induced porphyrins in tissue cells of a surgical margin;
an image sensor (190) provided in the distal tip (142) of the endoscope (140) of the system (100);
a first filter configured to detect and permit passage of optical signals, responsive to illumination of a target surface with the excitation light and configured to prevent passage of reflected excitation light and permit passage of emissions having wavelengths corresponding to autofluorescence emissions of tissue cells and fluorescence emissions of the induced porphyrins in tissue cells to the image sensor;
a white light source (165) located at the distal tip (142) of the endoscope (140) and configured to emit white light during white light imaging;
a second filter configured to detect and permit passage of optical signals, responsive to illumination of the target surface with the white light and having a wavelength in the visible light range, to the image sensor;
and
a processor configured to receive the detected fluorescent and white light optical signals and to output a representation of the target surface to the display (120) based on the detected optical signals.

2. System (100) according to claim 1, wherein the at least one excitation light source (160, 170) is configured to provide excitation light chosen to excite tissue autofluorescence emissions and as well as fluorescence emissions of induced porphyrins in tumor/cancer cells contained in a surgical margin of the excised tumor/tissue and/or in a surgical margin of a surgical bed from which tumor/cancer cells have been excised.

3. System (100) according to claim 1 or 2, wherein the at least one excitation light source (160) comprises an excitation light configured to provide excitation light having wavelength in the range of 350 nm - 450 nm and 550 nm - 600 nm, optionally 405 nm or 572nm.

4. System (100) according to claim 1 or 2 or 3, the at least one excitation light source (170) comprises an excitation light having a wavelength in the near infrared/infrared range, optionally having a wavelength of between about 750 nm and about 800 nm, for example 760 nm or 780 nm.

5. System (100) according to any one of the preceding claims, wherein the first filter is for fluorescence (FL) imaging and the second filter is for white light (WL) imaging and infrared (IR) imaging, wherein the system is configured to switch between different imaging filters based on desired imaging mode and the excitation light emitted by the handheld device.

6. System (100) according to claim 1, wherein positioned on an/the opposite, patient-facing side (125) of the system (100), the system includes handhold areas (130) configured to facilitate a user holding the system during imaging.

7. System (100) according to any one of the preceding claims comprising an optical printed circuit board (3600) including fluorescent LEDs (3604), infrared LEDs (3606), and white light LEDs (3608).

8. System (100) according to claim 10, wherein the optical printed circuit board (3600) also includes:
- an ambient light sensor (3610),
- a range finder, optionally a laser range finder, (3612), and
- a temperature sensor (3614).

9. System (100) according to claim 7 or 8, wherein the optical printed circuit board (3600) is a distal optical printed circuit board optionally positioned adjacent to the imaging sensor (190).

10. System (100) of any one of the preceding claims, further comprising a Wi-Fi and/or Bluetooth antenna; or
wherein the processor is configured to wirelessly transmit and/or receive data.

11. System (100) of any one of the preceding claims, further comprising a power source, wherein the power source is a battery.

12. System (100) of claim 4, wherein the system is configured to project infrared radiation onto the target surface and detect infrared radiation reflected from the target surface;
wherein the processor is further configured to generate a three-dimensional map of the target surface based on the detected reflected infrared radiation.

## Patentansprüche

1. Tragbares, handgehaltenes endoskopisches Bildgebungssystem (100), umfassend:
ein Endoskop (140);
einen Basiskörperabschnitt (110), wobei eine Vorderseite oder benutzerzugewandte Seite (115) des Basiskörperabschnitts (110) eine Anzeige (120) zum Anzeigen von durch das System (100) erfassten Bildern und Videos umfasst; wobei das Endoskop (140) auf einer gegenüberliegenden, patientenzugewandten Seite (125) des Systems (100) angeordnet ist;
mindestens eine Anregungslichtquelle (160, 170), die an einer distalen Spitze (142) des Endoskops (140) angeordnet und konfiguriert ist, um Autofluoreszenzemissionen von Gewebezellen und Fluoreszenzemissionen von induzierten Porphyrinen in Gewebezellen eines chirurgischen Randes anzuregen;
einen Bildsensor (190), der in der distalen Spitze (142) des Endoskops (140) des Systems (100) vorgesehen ist;
einen ersten Filter, der konfiguriert ist, um optische Signale als Reaktion auf die Beleuchtung einer Zieloberfläche mit dem Anregungslicht zu detektieren und deren Durchgang zu ermöglichen, und der konfiguriert ist, um den Durchgang von reflektiertem Anregungslicht zu verhindern und den Durchgang von Emissionen zu ermöglichen, deren Wellenlängen den Autofluoreszenzemissionen von Gewebezellen und den Fluoreszenzemissionen der induzierten Porphyrine in Gewebezellen entsprechen, zu dem Bildsensor;
eine Weißlichtquelle (165), die an der distalen Spitze (142) des Endoskops (140) angeordnet und konfiguriert ist, um während einer Weißlichtbildgebung Weißlicht zu emittieren;
einen zweiten Filter, der konfiguriert ist, um optische Signale als Reaktion auf die Beleuchtung der Zieloberfläche mit dem Weißlicht zu detektieren und deren Durchgang zu ermöglichen, wobei die optischen Signale eine Wellenlänge im sichtbaren Lichtbereich aufweisen, zu dem Bildsensor;
und
einen Prozessor, der konfiguriert ist, um die detektierten optischen Fluoreszenz- und Weißlichtsignale zu empfangen und auf Grundlage der detektierten optischen Signale eine Darstellung der Zieloberfläche auf der Anzeige (120) auszugeben.

2. System (100) nach Anspruch 1, wobei die mindestens eine Anregungslichtquelle (160, 170) konfiguriert ist, um ein Anregungslicht bereitzustellen, das ausgewählt ist, um Gewebe-Autofluoreszenzemissionen sowie Fluoreszenzemissionen von induzierten Porphyrinen in Tumor-/Krebszellen anzuregen, die in einem chirurgischen Rand des exzidierten Tumors/Gewebes und/oder in einem chirurgischen Rand eines chirurgischen Bettes enthalten sind, aus dem Tumor-/Krebszellen exzidiert wurden.

3. System (100) nach Anspruch 1 oder 2, wobei die mindestens eine Anregungslichtquelle (160) ein Anregungslicht umfasst, das konfiguriert ist, um ein Anregungslicht mit einer Wellenlänge im Bereich von 350 nm bis 450 nm und von 550 nm bis 600 nm bereitzustellen, gegebenenfalls 405 nm oder 572 nm.

4. System (100) nach Anspruch 1, 2 oder 3, wobei die mindestens eine Anregungslichtquelle (170) ein Anregungslicht mit einer Wellenlänge im Nahinfrarot-/Infrarotbereich umfasst, gegebenenfalls mit einer Wellenlänge zwischen etwa 750 nm und etwa 800 nm, beispielsweise 760 nm oder 780 nm.

5. System (100) nach einem der vorhergehenden Ansprüche, wobei der erste Filter für die Fluoreszenz(FL)-Bildgebung und der zweite Filter für die Weißlicht(WL)-Bildgebung und die Infrarot(IR)-Bildgebung vorgesehen ist, wobei das System konfiguriert ist, um zwischen verschiedenen Bildgebungsfiltern in Abhängigkeit von dem gewünschten Bildgebungsmodus und dem von der handgehaltenen Vorrichtung emittierten Anregungslicht umzuschalten.

6. System (100) nach Anspruch 1, wobei das System auf der gegenüberliegenden, patientenzugewandten Seite (125) Griffbereiche (130) umfasst, die konfiguriert sind, um einem Benutzer das Halten des Systems während der Bildgebung zu erleichtern.

7. System (100) nach einem der vorhergehenden Ansprüche, umfassend eine optische Leiterplatte (3600) mit Fluoreszenz-LEDs (3604), Infrarot-LEDs (3606) und Weißlicht-LEDs (3608).

8. System (100) nach Anspruch 10, wobei die optische Leiterplatte (3600) ferner umfasst:
- einen Umgebungslichtsensor (3610),
- einen Entfernungsmesser, gegebenenfalls einen Laser-Entfernungsmesser, (3612), und
- einen Temperatursensor (3614).

9. System (100) nach Anspruch 7 oder 8, wobei die optische Leiterplatte (3600) eine distale optische Leiterplatte ist, die gegebenenfalls angrenzend an den Bildgebungssensor (190) angeordnet ist.

10. System (100) nach einem der vorhergehenden Ansprüche, ferner umfassend eine Wi-Fi- und/oder Bluetooth-Antenne;
oder
wobei der Prozessor konfiguriert ist, um Daten drahtlos zu senden und/oder zu empfangen.

11. System (100) nach einem der vorhergehenden Ansprüche, ferner umfassend eine Energiequelle, wobei die Energiequelle eine Batterie ist.

12. System (100) nach Anspruch 4, wobei das System konfiguriert ist, um Infrarotstrahlung auf die Zieloberfläche zu projizieren und um von der Zieloberfläche reflektierte Infrarotstrahlung zu detektieren;
wobei der Prozessor ferner konfiguriert ist, um auf Grundlage der detektierten reflektierten Infrarotstrahlung eine dreidimensionale Karte der Zieloberfläche zu erzeugen.

## Revendications

1. Système d'imagerie endoscopique portable et tenu à la main (100), comprenant :
un endoscope (140) ;
une partie formant corps de base (110), une face avant ou face tournée vers l'utilisateur (115) de la partie formant corps de base (110) comportant un afficheur (120) destiné à afficher des images et des vidéos capturées par le système (100) ; dans lequel l'endoscope (140) est positionné sur une face opposée, tournée vers le patient (125), du système (100);
au moins une source de lumière d'excitation (160, 170) située au niveau d'une extrémité distale (142) de l'endoscope (140) et configurée pour exciter des émissions d'autofluorescence de cellules tissulaires et des émissions de fluorescence de porphyrines induites dans des cellules tissulaires d'une marge chirurgicale ;
un capteur d'image (190) prévu dans l'extrémité distale (142) de l'endoscope (140) du système (100);
un premier filtre configuré pour détecter et permettre le passage de signaux optiques, en réponse à l'illumination d'une surface cible par la lumière d'excitation, et configuré pour empêcher le passage de la lumière d'excitation réfléchie et pour permettre le passage d'émissions ayant des longueurs d'onde correspondant aux émissions d'autofluorescence de cellules tissulaires et aux émissions de fluorescence des porphyrines induites dans des cellules tissulaires, vers le capteur d'image ;
une source de lumière blanche (165) située au niveau de l'extrémité distale (142) de l'endoscope (140) et configurée pour émettre une lumière blanche pendant une imagerie en lumière blanche ;
un second filtre configuré pour détecter et permettre le passage de signaux optiques, en réponse à l'illumination de la surface cible par la lumière blanche et ayant une longueur d'onde dans la plage de la lumière visible, vers le capteur d'image ;
et
un processeur configuré pour recevoir les signaux optiques de fluorescence et de lumière blanche détectés et pour délivrer en sortie une représentation de la surface cible sur l'afficheur (120) sur la base des signaux optiques détectés.

2. Système (100) selon la revendication 1, dans lequel l'au moins une source de lumière d'excitation (160, 170) est configurée pour fournir une lumière d'excitation choisie pour exciter des émissions d'autofluorescence tissulaire ainsi que des émissions de fluorescence de porphyrines induites dans des cellules tumorales/cancéreuses contenues dans une marge chirurgicale de la tumeur/du tissu excisé(e) et/ou dans une marge chirurgicale d'un lit chirurgical duquel des cellules tumorales/cancéreuses ont été excisées.

3. Système (100) selon la revendication 1 ou 2, dans lequel l'au moins une source de lumière d'excitation (160) comprend une lumière d'excitation configurée pour fournir une lumière d'excitation ayant une longueur d'onde dans la plage de 350 nm à 450 nm et de 550 nm à 600 nm, éventuellement de 405 nm ou de 572 nm.

4. Système (100) selon la revendication 1, 2 ou 3, dans lequel l'au moins une source de lumière d'excitation (170) comprend une lumière d'excitation ayant une longueur d'onde dans la plage du proche infrarouge/infrarouge, éventuellement ayant une longueur d'onde comprise entre environ 750 nm et environ 800 nm, par exemple 760 nm ou 780 nm.

5. Système (100) selon l'une quelconque des revendications précédentes, dans lequel le premier filtre est destiné à l'imagerie de fluorescence (FL) et le second filtre est destiné à l'imagerie en lumière blanche (WL) et à l'imagerie infrarouge (IR), dans lequel le système est configuré pour commuter entre différents filtres d'imagerie en fonction du mode d'imagerie souhaité et de la lumière d'excitation émise par le dispositif tenu à la main.

6. Système (100) selon la revendication 1, dans lequel le système comprend, sur la face opposée tournée vers le patient (125), des zones de préhension (130) configurées pour faciliter la tenue du système par un utilisateur pendant l'imagerie.

7. Système (100) selon l'une quelconque des revendications précédentes, comprenant une carte de circuit imprimé optique (3600) comportant des DEL fluorescentes (3604), des DEL infrarouges (3606) et des DEL de lumière blanche (3608).

8. Système (100) selon la revendication 10, dans lequel la carte de circuit imprimé optique (3600) comprend en outre :
- un capteur de lumière ambiante (3610),
- un télémètre, éventuellement un télémètre laser, (3612), et
- un capteur de température (3614).

9. Système (100) selon la revendication 7 ou 8, dans lequel la carte de circuit imprimé optique (3600) est une carte de circuit imprimé optique distale, éventuellement positionnée de manière adjacente au capteur d'imagerie (190).

10. Système (100) selon l'une quelconque des revendications précédentes, comprenant en outre une antenne Wi-Fi et/ou Bluetooth ;
ou
dans lequel le processeur est configuré pour transmettre et/ou recevoir des données sans fil.

11. Système (100) selon l'une quelconque des revendications précédentes, comprenant en outre une source d'alimentation, dans lequel la source d'alimentation est une batterie.

12. Système (100) selon la revendication 4, dans lequel le système est configuré pour projeter un rayonnement infrarouge sur la surface cible et pour détecter un rayonnement infrarouge réfléchi par la surface cible ;
dans lequel le processeur est en outre configuré pour générer une carte tridimensionnelle de la surface cible sur la base du rayonnement infrarouge réfléchi détecté.
